# EUROPEAN PATENT APPLICATION

(11) **EP 2 676 962 A1**
(43) Date of publication of application: **25.12.2013**
(21) Application number: 13172944.4
(22) Date of filing: 20.06.2013
(51) Int. Cl.: C07F 9/40, C12Q 1/37, G01N 33/573

(54) **Derivatives of 1-aminoalkylphosphonate diaryl esters, method of 1-aminoalkylphosphonate diaryl ester derivatives preparation and their application**

(30) Priority: 21.06.2012 PL 39961312
(71) Applicant: Universität Ulm, 89081 Ulm (DE); Wroclaw University of Technology, 50-370 Wroclaw (PL)
(72) Inventor: Sienczyk, Marcin, 50-270 Wroclaw (PL); Grzywa, Renata, 50-270 Wroclaw (PL); Pietrusewicz, Ewa, 50-270 Wroclaw (PL); Oleksyszyn, Józef, 50-270 Wroclaw (PL); Winiarski, Lukasz, 50-270 Wroclaw (PL); Burster, Timo, Ulm (DE); Böhm, Otto, Ulm (DE)
(74) Representative: Pfenning, Meinig & Partner GbR

(57) **Abstract**

The subject of the invention relates to 1-aminoalkylphosphonate diaryl esters of formula I, in which Bt represents biotin, W is a hydrocarbon linker chain of 5 to 10 carbon atoms, Y is the amino acid residues in an amount of 0 to 4, R is a substituent corresponding to the side chain of valine, alanine, leucine, phenylalanine, 4-guanidinephenylglycine, or 2-aminobutyric acid (Abu), X is the hydrogen or a compound selected from the group consisting of mercaptomethyl (S-methyl), methoxy (O-methyl), or carboxymethyl, and their use as inhibitors of serine proteases, and the low molecular weight molecular probes to detect the catalytically active forms of the enzymes.

A process for preparing 1-aminoalkylphosphonate diaryl esters of formula I, in which Bt represents biotin, W is a hydrocarbon linker chain of 5 to 10 carbon atoms, Y is the amino acid residues in an amount of 0 to 4, R is a substituent corresponding to the side chain of valine, alanine, leucine, phenylalanine, 4-guanidinephenylglycine, or 2-aminobutyric acid (Abu), X is the hydrogen or a compound selected from the group consisting of mercaptomethyl (S-methyl), methoxy (O-methyl), or carboxymethyl relies on application of triaryl phosphite, obtained from the phenol substituted at *para* position phosphorus (III) chloride in refluxing acetonitrile and is subjected to amidoalkylation reaction with benzyl carbamate and an aldehyde such as acetaldehyde, propionic aldehyde, *iso*-butyric aldehyde, phenylacetic aldehyde, p-nitrobenzoic aldehyde and isovaleric aldehyde, followed by the synthesis of hydrobromide salts of 1-aminoalkylphosphonate diaryl esters, in which the benzyloxycarbonyl (Cbz) protective group is removed, and in the next step is reacted with an amino acid containing protected α-amino group in the presence of a coupling reagent. The resulting phosphonic dipeptide containing tert-butoxycarbonyl (t-Boc) protective group is subjected to deprotection of α-amino group and obtained dipeptidyl 1-aminoalkylphosphonate diaryl ester is coupled with another amino acid with protected α-amino group, biotin or a derivative thereof in a manner analogous to that described above.

## Description

The subject of invention relates to diaryl esters of 1-aminoalkylphosphonates, a method for preparation of 1-aminoalkylphosphonate diaryl ester derivatives and their application as serine proteases inhibitors as well as the low molecular weight molecular probes to detect the catalytically active forms of the enzymes that can be used in medical diagnostics.

The publication of J. Oleksyszyn, Subotkowska L., Mastalerz P., Synthesis, 1979, 985, J. Oleksyszyn, JC Powers, Biochemistry, 1991, 30, 485 describes the synthesis of diphenyl esters of 1-aminoalkylphosphonates as structural analogues of amino acids that are capable of proteolytic enzymes of the family of serine proteases inhibition. The publications Boduszek B., Brown AD, Powers JC, J. Enzyme Inhib. 1994, 8, 147-158; Sieńczyk M., Oleksyszyn J., Bioorg Med Chem Lett, 2006, 16, 2886; Joossens J., Ali OM, E1-Sayed I, Surpateanu G., Van der Veken P., Lambeir AM, Setyono-Han B., Foekens JA, Schneider, A., Schmalix W., Haemers A., K. Augustyns, J Med Chem., 2007, 50, 6638; Sieńczyk M., D. Podgorski, Blażejewska A., Kulbacka J., Saczko J., Oleksyszyn J., Bioorg Med Chem., 2011, 19, 1277; Sieńczyk M., L. Winiarski, Kasperkiewicz P., Psurski M., J. Wietrzyk, J. Oleksyszyn, Bioorg Med Chem Lett, 2011, 21, 1310 describe several examples of 1-aminoalkylphosphonate diaryl ester derivatives which contain substituted phenyl ester within their structure.

Cathepsins are important enzymes that participate in the antigens processing within the major histocompatibility class II (MHC II) pathway. Three classes of proteases, cysteine, aspartyl, and serine proteases, located in the endocytic compartment are responsible for the proteolytic potential. Combined action of these proteases results in the production of antigenic peptides, which are fragments derived from antigens, that when combined with the MHC class II proteins are presented to CD4 T-cell receptors (CD4⁺). However, the detection of serine protease cathepsin G (CatG) in B cells and dendritic cells is constrained by the need to use large amounts of cell lysates. Using commercially available low molecular weight molecular probes for the detection of enzymatically active cathepsin G it is necessary to use approximately 20µg of cell lysate to determine its presence.

Derivatives of 1-aminoalkylphosphonate diaryl esters, which are the subject of the invention, which have specific properties to irreversibly inhibit serine proteases and are low molecular weight molecular probes, which are used for detecting and imaging an enzymatically active form of this class of enzymes, have not yet been described in the literature. Biotin molecule is used as a specific tag that allows to detect the protease-inhibitor complex. Probes belonging to this class of compounds that have been described in the scientific publications previously do not include the presence/introduction of any substituents within the aromatic ester rings, so their inhibitory properties are lower than the derivatives of the invention.

The invention relates to derivatives of 1-aminoalkylphosphonate diaryl esters of the general formula I in which Bt is biotin, W is a linker comprising a hydrocarbon of 5 to 10 carbon atoms, Y is an amino acid residue in an amount of 0 to 4, R is a substituent corresponding to the side chain of valine, alanine, leucine, phenylalanine, 4-guanidinephenylglicine or 2-aminobutyric acid (Abu), X is hydrogen or a compound selected from the group consisting of: thiomethyl (S-methyl), methoxy (O-methyl), or carboxymethyl.

Preferably, derivatives of 1-aminoalkylphosphonate diaryl esters are mixtures of diastereomers.

A process for preparing diaryl ester derivatives of 1-aminoalkylphosphonic acids of the general formula I in which Bt is biotin, W is a linker comprising a hydrocarbon of 5 to 10 carbon atoms, Y is an amino acid residue in an amount of 0 to 4, R is a substituent corresponding to the side chain of valine, alanine, leucine, phenylalanine, 4-guanidinephenylglicine or 2-aminobutyric acid (Abu), X is hydrogen or a compound selected from the group consisting of: thiomethyl (S-methyl), methoxy (O-methyl), or carboxymethyl is, that triaryl phosphite, obtained from the para-substituted phenol and phosphorus chloride (III) in acetonitrile, and subjected to amidoalkylation reaction using benzyl carbamate, triaryl phosphite and an aldehyde such as acetaldehyde, propionic, isobutyric, phenylacetic, p-nitrobenzoic, or isovaleric. Then the synthesis of hydrobromide salts of 1-aminoalkylphosphonate diaryl esters is carried out in order to remove the protecting benzyloxycarbonyl (Cbz) group followed by the conjugation with the α-N-Boc-protected amino acid in the presence of a coupling reagent. From the resulting phosphonic dipeptide the t-butoxycarbonyl (t-Boc) group is removed leading to phosphonic dipeptide containing free N-terminal amine group, which is reacted in the presence of a coupling reagent in a manner analogous to that described above, with next amino acid containing protected α-amino group, or biotin or a derivative thereof.

Preferably, N,N,N',N' -tetramethyl-O-(1*H*-benzotriazol-1-yl)uronium hexafluorophosphate (HBTU) in the presence of N, N'-diisopropylethylamine (DIEA) is used as the coupling reagent.

The application of derivatives of 1-aminoalkylphosphonate diaryl esters of the general formula I in which Bt is biotin, W is a linker comprising a hydrocarbon of 5 to 10 carbon atoms, Y is an amino acid residue in an amount of 0 to 4, R is a substituent corresponding to the side chain of valine, alanine, leucine, phenylalanine, 4-guanidinephenylglicine or 2-aminobutyric acid (Abu), X is hydrogen or a compound selected from the group consisting of: thiomethyl (S-methyl), methoxy (O-methyl), or carboxymethyl as inhibitors of serine proteases.

The application of derivatives of 1-aminoalkylphosphonate diaryl esters of the general formula I in which Bt is biotin, W is a linker comprising a hydrocarbon of 5 to 10 carbon atoms, Y is an amino acid residue in an amount of 0 to 4, R is a substituent corresponding to the side chain of valine, alanine, leucine, phenylalanine, 4-guanidinephenylglicine or 2-aminobutyric acid (Abu), X is hydrogen or a compound selected from the group consisting of: thiomethyl (S-methyl), methoxy (O-methyl), or carboxymethyl as the low molecular weight probes for the detection of catalytically active serine proteases forms.

The diagnostic application of 1-aminoalkylphosphonate diaryl ester derivatives is based on their irreversible binding to the active site of the target serine protease only, which eliminates the false positive results originated from the presence of catalytically inactive proteases in the tested sample, in contrast to the assays based on antibodies since antibodies are not able to discriminate active vs. inactive enzyme. Another advantage is the high specificity of action toward serine proteases and the lack of reaction with other classes of proteases such as cysteine, threonine or metalloproteases. After binding to the active site of the target protease a covalent bond that is being formed is very stable and is not cleaved even after boiling in the presence of sodium dodecyl sulfate and β-mercaptoethanol, thereby allowing imaging of complexes by western blotting technique. For this purpose, streptavidin-horseradish peroxidase conjugate is used as a detection agent to visualize enzyme-inhibitor complex. The sensitivity of the method allows for the detection of active forms of the target enzymes from several to several tens of ng, both in the biological material (cells of human peripheral blood mononuclear cells, PBMC) or bacterial cell cultures (culture of *Bacillus subtilis*) or in the purified proteins (in order to confirm the activity of isolated enzymes). Moreover, the detection capability is greater than the use of enzyme-specific antibodies and is significantly more cost-efficient than the immunodetection methods.

The object of the invention is shown in the examples of 1-aminoalkylphosphonate diaryl ester derivatives preparation, measurement of their inhibitory properties and the application in the detection of enzymatically active forms of the selected proteases.

### Example 1

The method for preparation of bis(4-(methylthio)phenyl)-2-methyl-1-(1-(3-methyl-2-(5-(2-oxohexahydro-1H-thieno[3,4-d]imidazol-4-yl)pentanamido)butanoyl)pyrrolidine-2-carboxamido)propylphosphonate [Bt-Val-Pro-Val^{P}(O-C₆H₄-4-S-CH₃)₂] represented by the formula 1.

### Step 1. Synthesis of tri-(4-thiomethyl)phenyl phosphite.

In the round-bottom flask equipped with a magnetic stirrer 4,2g (30mmol) of 4-thiomethylphenol is dissolved in acetonitrile (20ml) followed by the addition of phosphorus (III) chloride (0,87ml, 10mmol). The reaction mixture is then refluxed for 6h. The volatile elements of the mixture are removed under reduced pressure and resulting product obtained as pale yellow oil is used without purification for the synthesis of di-4-thiomethylphenyl ester of *N*-(benzyloxycarbonyl-1-amino)-2-methyl-propanephosphonic acid.

*Step 2. Synthesis of di-4-thiomethylphenyl ester of N-(benzyloxycarbonyl-1-amino)-2-methyl-propanephosphonic acid.*

### Into the flask containing tri-(4-thiomethyl)phenyl phosphite obtained in step 1, 1,51g (10mmol) of benzyl carbamate and 1,1ml (12mmol) of iso-butyl aldehyde are added followed by the addition of glacial acetic acid (10 ml). The reaction mixture is heated at 90°C for 3h and the volatile components of the mixture are removed under reduced pressure. The product obtained as yellow oil is dissolved in 100-150ml of methanol and stored at -20°C for crystallization (24h). Crystallized product is collected by suction and air-dried obtaining 1,92g (36% yield). Empirical Formula: C₂₆H₃₀NO₅PS₂. Molecular weight: 531,62g/mol. ³¹P NMR (121MHz, CDCl₃, δ[ppm]) :δ 19.45 (s); ¹H NMR (300 MHz, CDCl₃, ppm): δ 7.41-6.99 (m, 13H), 5.19-5.06 (m, 3H), 4.65-4.44 (m, 1H), 2.45 (s, 3H, SCH₃), 2.44 (s, 3H, SCH₃), 1.63-1.50 (m, 3H). MS m/z 503.10/504.18 (M+H)⁺. mp: 103°C.

### Step 3. Synthesis of di-4-thiomethylphenyl ester of 1-amino-2-methylpropanephosphonic acid hydrobromide salt.

Into the round-bottom flask di-4-thiomethylphenyl ester of N-(benzyloxycarbonyl-1-amino)-2-methyl-propanephosphonic acid (2,00g, 3,76 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,70 g (95% yield) of di-4-thiomethylphenyl ester of 1-amino-2-methyl-propanephosphonic acid hydrobromide salt. Empirical Formula: C₁₈H₂₅BrNO₃PS₂. Molecular weight: 477,02g/mol. ³¹P NMR (121MHz, DMSO-*d₆*, δ[ppm]) :δ 15,87 (s); ¹H NMR (300 MHz, DMSO-*d₆*, ppm): δ 1,14 (dd, *J*=6,9/11,6; 6H, 2xCH₃), 2,35 (m, 1H, CH), 4,1 (m, 1H, CHP), 7,07-7,32 (m, 8H, Ar-H), 8,66 (s, 3H, NH₃⁺). mp: 147°C.

### Step 4. Synthesis of dipeptidyl derivative of di-4-thiomethylphenyl ester of 1-amino-2-methyl-propanephosphonic acid.

Into the round-bottom flask 0,95g (2 mmol) of di-4-thiomethylphenyl ester of 1-amino-2-methyl-propanephosphonic acid hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 1,07g (90%). Empirical Formula: C₂₈H₃₉O₆N₂PS₂. Molecular weight: 594,20 g/mol. ³¹P NMR (121MHz, CDCl₃, δ[ppm]) :δ 18,59 (s). ¹H NMR (300 MHz, CDCl₃, ppm): δ 7,24-7,00 (m, 8H), 4,80-4,63 (m, 1H), 4,43-4,27 (m, 1H), 3,52-3,24 (m, 2H), 2,46-2,43 (m, 6H), 2,33-2,06 (m, 2H), 2,03-1,86 (m, 2H), 1,86-1-74 (m, 1H), 1,72-1,59 (m, 1H), 1,44 (s, 9H), 1,15-0,96 (m, 6H). R_{f} = 0,41 (CHCl₃:AcOEt; 4:1, *v*/*v*).

### Step 5. Deprotection of t-Boc group in dipeptidyl derivative of di-4-thiomethylphenyl ester of 1-amino-2-methyl-propanephosphonic acid.

Dipeptidyl derivative of di-4-thiomethylphenyl ester of 1-amino-2-methylpropanephosphonic acid is placed in the flask (1,0 g, 1,68 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,98g which is used without purification in the next step for the synthesis of tripeptidyl derivative of di-4-thiomethylphenyl ester of 1-amino-2-methyl-propanephosphonic acid.

### Step 6. Synthesis of tripeptidyl derivative of di-4-thiomethylphenyl ester of 1-amino-2-methyl-propanephosphonic acid.

Into the product obtained in Step 5 - TFA*Pro-Val^{P}(OC₆H₄-4-S-CH₃)₂ (0,98g; 1,66mmol) 0,37g (1,66mmol) of Boc-Val-OH is added. After 2 minutes from complete dissolving in acetonitrile (15ml), 0,55ml of triethylamine (4mmol) is added. After 10 minutes 0,68g (1.8mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 1,04g (90%). Empirical Formula: C₃₃H₄₈N₃O₇PS₂. Molecular weight: 693,85g/mol. ³¹P NMR (CDCl₃, δ [ppm]): 17.94 (s, 47%), 17.56 (s, 53%). ¹H NMR (CDCl₃, δ[ppm]) :δ 7,25-7,04 (m, 8H), 5,28-5,10 (m, 1H), 4,78-4,68 (m, 1H), 4,67-4,45 (m, 1H), 4,35-4,20 (m, 1H), 3,84-3.43 (m, 2H), 2,49-2.43 (m, 6H), 2,43-1,76 (m, 7H,), 1,49-1,36 (m, 9H), 1,15-0,82 (m, 12H). R_{f} = 0,21 (CHCl₃: AcOEt; 4:1, v/v).

### Step 7. Deprotection of t-Boc group in tripeptidyl derivative of di-4-thiomethylphenyl ester of 1-amino-2-methyl-propanephosphonic acid.

Tripeptidyl derivative of di-4-thiomethylphenyl ester of 1-amino-2-methylpropanephosphonic acid is placed in the flask (1,0 g, 1,44 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,94g which is used without purification in the next step for the synthesis of biotinylated tripeptidyl derivative of di-4-thiomethylphenyl ester of 1-amino-2-methyl-propanephosphonic acid.

### Step 8. Synthesis of biotinylated tripeptidyl derivative of di-4-thiomethylphenyl ester of 1-amino-2-methyl-propanephosphonic acid.

Product obtained in step 7 (TFA*Val-Pro-Val^{P}(OC₆H₄-4-S-CH₃)₂ (0,94g, 1,36mmol) is dissolved in N-methylpyrrolidone (10ml) followed by the addition of biotin (0,33g, 1,36mmol). Next, N,N'-diisopropylamine (DIEA) is added in the amount of 0,48ml (3,44mmol). After complete dissolving 0,57g (1,50mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,54g, (38%). Empirical Formula: C₃₈H₅₄N₅O₇PS₃. Molecular weight: 820,03g/mol. Retention time (HPLC, Alltech Econosphere C-18 10U column, 250x22mm, gradient 5%-95% MeCN/45min): 23,44 min. HRMS m/z 842,2820/842,2745 (M+Na)⁺.

### Example 2

The method for preparation of diphenyl 2-methyl-1-(1-(3-methyl-2-(5-(2-oxohexahydro-1H-thieno[3,4-d]imidazol-4-yl)pentanamido)butanoyl)pyrrolidine-2-carboxamido)propylphosphonate [Bt-Val-Pro-Val^{P}(O-C₆H₅)₂, represented by the formula 2.

### Step 1. Synthesis of diphenyl ester of N-(benzyloxycarbonyl-1-amino)-2-methyl-propanephosphonic acid.

Into the round-bottom flask containing triphenyl phosphite (3,10g, 10mmol) benzyl carbamate, (1,51g, 10mmol) and isobutyraldehyde 1,1ml (10,5mmol) are added followed by the addition of glacial acetic acid (10 ml). The reaction was performed at 90°C for 4h. The volatile elements of the reaction mixture were removed in vacuum and the resulting oil was dissolved in methanol (100-150ml). The solution is placed at -20°C for 24 h to complete crystallization. The product is collected by suction and air-dried to obtain 2,11g (48% yield). Empirical Formula: C₂₄H₂₆NO₅P. Molecular weight: 439,44g/mol. ³¹P NMR (121MHz, CDCl₃, δ[ppm]) :δ 17,81 (s); ¹H NMR (300 MHz, CDCl₃, ppm): δ 1,11 (d, J=6,8 Hz, 6H), 4,41-4,47 (m, 1H), 4,72 (s, 1H), 5,13 (d, J=10,6 Hz, 1H), 5,27 (d, J=4,1 Hz, 2H), 7,06-7,40 (m, 15H). mp: 107°C.

### Step 2. Synthesis of diphenyl ester of 1-amino-2-methylpropanephosphonic acid hydrobromide salt.

Into the round-bottom flask diphenyl ester of N-(benzyloxycarbonyl-1-amino)-2-methyl-propanephosphonic acid (2,00g, 4.55 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,34 g (76% yield) of diphenyl ester of 1-amino-2-methyl-propanephosphonic acid hydrobromide salt. Empirical Formula: C₁₆H₂₁BrNO₃P. Molecular weight: 386,22 g/mol. ³¹P NMR (121MHz, DMSO-*d₆*, δ [ppm]): δ 14,74 (s); ¹H NMR (300 MHz, DMSO-*d₆*, δ [ppm]): δ 1,20 (dd, *J*=7,0/22,9 Hz, 6H), 2,39-2,49 (m, 1H), 4,15 (dd, J=4,8/15,4 Hz, 1H), 7,05-7,46 (m, 10H), 8,74 (s, 3H, NH₃⁺). mp: 171°C.

### Step 3. Synthesis of dipeptidyl derivative of diphenyl ester of 1-amino-2-methyl-propanephosphonic acid.

Into the round-bottom flask 0,79g (2 mmol) of diphenyl ester of 1-amino-2-methyl-propanephosphonic acid hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0.95g (95%). Empirical Formula: C₂₆H₃₅O6N₂P. Molecular weight: 502,54 g/mol. ³¹P NMR (121MHz, CDCl₃, δ[ppm]) :δ 17,45 (s); ¹H NMR (300 MHz, CDCl₃, ppm): δ 7,36-7.08 (m, 10H), 4,84-4,63 (m, 1H), 4,45-4,26 (m, 1H), 3,57-3,23 (m, 2H), 2,53-2,33 (m, 2H), 2,31-2,04 (m, 2H), 1,98-1,87 (m, 1H), 1,83-1,64 (m, 1H), 1,45 (s, 9H), 1,16-0,98 (m, 6H). R_{f} = 0,56 (CHCl₃:AcOEt; 4:1, v/v).

### Step 4. Deprotection of t-Boc group in dipeptidyl derivative of diphenyl ester of 1-amino-2-methyl-propanephosphonic acid.

Dipeptidyl derivative of diphenyl ester of 1-amino-2-methyl-propanephosphonic acid is placed in the flask (2.05 g, 2 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 1.88g which is used without purification in the next step for the synthesis of tripeptidyl derivative of di-4-thiomethylphenyl ester of 1-amino-2-methylpropanephosphonic acid.

### Step 5. Synthesis of tripeptidyl derivative of diphenyl ester of 1-amino-2-methyl-propanephosphonic acid.

Into the product obtained in Step 4 - TFA*Pro-Val^{P}(OC₆H₄)₂ (0,83g; 1,66mmol) 0,37g (1,66mmol) of Boc-Val-OH is added. After 2 minutes from complete dissolving in acetonitrile (15ml), 0,55ml of triethylamine (4mmol) is added. After 10 minutes 0,68g (1.8mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0.92g (92%). Empirical Formula: C₃₁H₄₄N₃O₇P. Molecular weight: 601,67 g/mol. ³¹P NMR (CDCl₃, δ [ppm]): 17,40 (s, 46%), 17,07 (s, 54%). ¹H NMR (CDCl₃, δ[ppm]): 7,32-7,09 (m, 10H), 5,39-5,15 (m, 1H), 4,82-4,70 (m, 1H), 4,70-4,45 (m, 1H), 4,34-4,18 (m, 1H), 3,84-3,41 (m, 2H), 2,50-1,75 (m, 7H), 1,48-1,38 (m, 9H), 1,15-0,81 (m, 12H). HRMS *m*/*z* 624,2815/624,2795 (M+Na⁺). R_{f}= 0,19 (CHCl₃:AcOEt; 4:1, v/v).

### Step 6. Deprotection of t-Boc group in tripeptidyl derivative of diphenyl ester of 1-amino-2-methyl-propanephosphonic acid.

Tripeptidyl derivative of diphenyl ester of 1-amino-2-methyl-propanephosphonic acid is placed in the flask (1,0 g, 1,66 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,88g which is used without purification in the next step for the synthesis of biotinylated tripeptidyl derivative of diphenyl ester of 1-amino-2-methyl-propanephosphonic acid.

### Step 7. Synthesis of biotinylated tripeptidyl derivative of di-4-thiomethylphenyl ester of 1-amino-2-methyl-propanephosphonic acid.

Product obtained in step 6 (TFA*Val-Pro-Val^{P}(OC₆H₄)₂ (0,82g, 1,36mmol) is dissolved in N-methylpyrrolidone (10ml) followed by the addition of biotin (0,33g, 1,36mmol). Next, N,N'-diisopropylamine (DIEA) is added in the amount of 0,48ml (3,44mmol). After complete dissolving 0,57g (1,50mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,29g, (29%). Empirical Formula: C₃₆H₅₀N₅O₇PS. Molecular weight: 727,85 g/mol. Retention time (HPLC, Alltech Econosphere C-18 10U column, 250x22mm, gradient 5%-95% MeCN/45min): 18,81 min. HRMS m/z 750,3066/750,1553 (M+Na)⁺.

### Example 3

The method for preparation of diphenyl 1-(1-(3-methyl-2-(5-(2-oxohexahydro-1H-thieno[3,4-d]imidazol-4-yl)pentanamido)butanoyl)pyrrolidine-2-carboxamido)-2-phenylethylphosphonate [Bt-Val-Pro-Phe^{P}(O-C₆H₅)₂, represented by the formula 3.

### Step 1. Synthesis of diphenyl ester of N-(benzyloxycarbonyl-1-amino)-2-phenyl-ethanephosphonic acid.

Into the round-bottom flask containing triphenyl phosphite (3,10g, 10mmol) benzyl carbamate, (1,51g, 10mmol) and phenylacetaldehyde 1,45ml (12mmol) are added followed by the addition of glacial acetic acid (10 ml). The reaction was performed at 90°C for 4h. The volatile elements of the reaction mixture were removed in vacuum and the resulting oil was dissolved in methanol (100-150ml). The solution is placed at -20°C for 24 h to complete crystallization. The product is collected by suction and air-dried to obtain 2,73g (56% yield). Empirical Formula: C₂₈H₂₆NO₅P. Molecular weight: 487,48 g/mol. ³¹P NMR (121MHz, CDCl₃, δ[ppm]) :δ 18,46 (s); ¹H NMR (300 MHz, CDCl₃, ppm): δ 2,98-3,48 (m, 2H), 4,82-4,85 (m, 1H), 4,88 (s, 2H), 5,15 (d, *J =* 10.17 Hz, 1H), 7,06-7,29 (m, 20H). mp: 124,5-125,5°C.

### Step 2. Synthesis of diphenyl ester of 1-amino-2 phenyl-ethanephosphonic acid hydrobromide salt.

Into the round-bottom flask diphenyl ester of N-(benzyloxycarbonyl-1-amino)-2-phenyl-ethanephosphonic acid (2,00g, 4.10 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,67 g (94% yield) of diphenyl ester of 1-amino-2-phenyl-ethanephosphonic acid hydrobromide salt. Empirical Formula: C₂₀H₂₁BrNO₃P. Molecular weight: 434,26 g/mol. ³¹P NMR (121 MHz, DMSO-*d₆*, δ[ppm]) :δ 15,31 (s); ¹H NMR (300 MHz, DMSO-*d₆*, ppm): δ 2,48-2,49 (m, 2H), 4,50-4,55 (m, 1H), 7,04-7,41 (m, 15H), 8,78 (s, 3H).

### Step 3. Synthesis of dipeptidyl derivative of diphenyl ester of 1-amino-2-phenyl-ethanephosphonic acid.

Into the round-bottom flask 0,86g (2 mmol) of diphenyl ester of 1-amino-2-methyl-propanephosphonic acid hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4: 1) as the eluent. The product is obtained as colorless oil, 0.70g (64%). Empirical Formula: C₃₀H₃₅O₆N₂P. Molecular weight: 550,58 g/mol. ³¹P NMR (121MHz, CDCl₃, δ[ppm]) :δ 18,56 (s, 49%), 18.29 (s, 51%). ¹H NMR (300 MHz, CDCl₃, ppm): δ 1,22-1,25 (m, 6H), 1,36-1,98 (m, 6H, 3×CH₂), 3.41-3.43 (m, 3H, CH₂, CH), 4.19-4.21 (m, 1H, NH), 5.18-5.19 (m, 1H, CHP), 7.04-7.67 (m. 15H, aromat.).

### Step 4. Deprotection of t-Boc group in dipeptidyl derivative of diphenyl ester of 1-amino-2 phenyl-ethanephosphonic acid.

Dipeptidyl derivative of diphenyl ester of 1-amino-2-phenyl-ethanephosphonic acid is placed in the flask (0.92 g, 1.68 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil (0.69g) which is used without purification in the next step for the synthesis of tripeptidyl derivative of diphenyl ester of 1-amino-2-phenyl-ethanephosphonic acid.

### Step 5. Synthesis of tripeptidyl derivative of diphenyl ester of 1-amino-2-phenyl-ethanephosphonic acid.

Into the product obtained in Step 4 - TFA*Pro-Phe^{P}(OC₆H₄)₂ (0,91g; 1,66mmol) 0,37g (1,66mmol) of Boc-Val-OH is added. After 2 minutes from complete dissolving in acetonitrile (15ml), 0,55ml of triethylamine (4mmol) is added. After 10 minutes 0,68g (1.8mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4: 1) as the eluent. The product is obtained as colorless oil, 0.25g (23%). Empirical Formula: C₃₅H₄₄N₃O₇P. Molecular weight: 649,71 g/mol. ³¹P NMR (CDCl₃, δ [ppm]): δ 17,35 (s). ¹H NMR (CDCl₃, δ[ppm]) : δ 0,68-0,72 (m, 6H), 1,20 (s, 9H), 1,50-1,79 (m, 6H), 2,83-2,86 (m, 1H), 3,14-3,39 (m, 3H), 3,99-4,02 (m, 1H), 5,04-5,06 (m, 1H), 5,16 (s, 1H), 6,87-7,07 (m, 15H).

### Step 6. Deprotection of t-Boc group in tripeptidyl derivative of diphenyl ester of 1-amino-2 phenyl-ethanephosphonic acid.

Tripeptidyl derivative of diphenyl ester of 1-amino-2-phenyl-ethanephosphonic acid is placed in the flask (0.96 g, 1,48 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil (0,77g) which is used without purification in the next step for the synthesis of biotinylated tripeptidyl derivative of diphenyl ester of 1-amino-2-phenyl-ethanephosphonic acid.

### Step 7. Synthesis of biotinylated tripeptidyl derivative of diphenyl ester of 1-amino-2-phenyl-ethanephosphonic acid.

Product obtained in step 6 (TFA*Val-Pro-Phe^{P}(OC₆H₄)₂ (0,88g, 1,36mmol) is dissolved in N-methylpyrrolidone (10ml) followed by the addition of biotin (0,33g, 1,36mmol). Next, N,N'-diisopropylamine (DIEA) is added in the amount of 0,48ml (3,44mmol). After complete dissolving 0,57g (1,50mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,42g, (36%). Empirical Formula: C₄₀H₅₀N₅O₇PS. Molecular weight: 775,89 g/mol. Retention time (HPLC, Alltech Econosphere C-18 10U column, 250x22mm, gradient 5%-95% MeCN/45min): 26,36 min. HRMS m/z 776,3247/776,1211 (M+H)⁺.

### Example 4

The method for preparation of bis(4-(methylthio)phenyl) 1-(1-(3-methyl-2-(5-(2-oxohexahydro-1H-thieno[3,4-d]imidazol-4-yl)pentanamido)butanoyl) pyrrolidine-2-carboxamido)ethylphosphonate [Bt-Val-Pro-Ala^{P}(O-C₆H₄-4-S-CH₃)₂] represented by the formula 4.

### Step 1. Synthesis of tri-(4-thiomethyl)phenyl phosphite.

In the round-bottom flask equipped with a magnetic stirrer 4,2g (30mmol) of 4-thiomethylphenol is dissolved in acetonitrile (20ml) followed by the addition of phosphorus (III) chloride (0,87ml, 10mmol). The reaction mixture is then refluxed for 6h. The volatile elements of the mixture are removed under reduced pressure and resulting product obtained as pale yellow oil is used without purification for the synthesis of di-4-thiomethylphenyl ester of *N*-(benzyloxycarbonyl-1-amino)-ethanephosphonic acid.

### Step 2. Synthesis of di-4-thiomethylphenyl ester of N-(benzyloxycarbonyl-1-amino)-ethanephosphonic acid.

Into the flask containing tri-(4-thiomethyl)phenyl phosphite obtained in step 1, 1,51g (10mmol) of benzyl carbamate and 1,1ml (12mmol) of acetaldehyde are added followed by the addition of glacial acetic acid (10 ml). The reaction mixture is heated at 90°C for 3h and the volatile components of the mixture are removed under reduced pressure. The product obtained as yellow oil is dissolved in 100-150ml of methanol and stored at -20°C for crystallization (24h). Crystallized product is collected by suction and air-dried obtaining 3.07g (61% yield). Empirical Formula: C₂₄H₂₆NO₅PS₂. Molecular weight: 503,57 g/mol. ³¹P NMR (121MHz, CDCl₃, δ[ppm]) :δ 19,48 (s); ¹H NMR (300 MHz, CDCl₃, ppm): δ 1,17-1,22 (d, *J* = 9,0 Hz, 3H), 2,45 (s, 6H), 4,49-4,63 (dd, *J* = 9,0/15,0 Hz ,1H), 5,08-5,17 (m, 3H), 6,99-7,35 (m, 13H). mp: 103°C. R_{f} = 0,65 (CHCl₃:AcOEt, 4:1, v/v).

### Step 3. Synthesis of di-4-thiomethylphenyl ester of 1-amino-ethanephosphonic acid hydrobromide salt.

Into the round-bottom flask di-4-thiomethylphenyl ester of N-(benzyloxycarbonyl-1-amino)-ethanephosphonic acid (2,00g, 3,97 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,70 g (95% yield) of di-4-thiomethylphenyl ester of 1-amino-ethanephosphonic acid hydrobromide salt. Empirical Formula: C₁₆H₂₁BrNO₃PS₂. Molecular weight: 450,35 g/mol. ³¹P NMR (121MHz, DMSO-*d₆*, δ[ppm]) :δ 16,85 (s); ¹H NMR (300 MHz, DMSO-*d₆*, ppm): δ 1,49-1,59 (m, 3H), 2,48 (s, 6H), 4,21-4,30 (m, 1H), 7,14-7,32 (m, 8H), 8,76 (s, 3H). mp: 176°C. R_{f} = 0,31 (CHCl₃:AcOEt, 4:1, v/v).

### Step 4. Synthesis of dipeptidyl derivative of di-4-thiomethylphenyl ester of 1-amino-ethanephosphonic acid.

Into the round-bottom flask 0,9g (2 mmol) of di-4-thiomethylphenyl ester of 1-amino-2-methyl-propanephosphonic acid hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0.88g (78%). Empirical Formula: C₂₆H₃₅O₆N₂PS₂. Molecular weight: 566,67 g/mol. ³¹P NMR (121MHz, CDCl₃, δ[ppm]) :δ 19,61 (s); ¹H NMR (300 MHz, CDCl₃, ppm): δ 1,19-1,24 (d, *J =* 15,0 Hz, 3H), 1,40 (s, 9H), 1,46-1,89 (m, 5H), 2,00 (s, 6H), 3,31-3,40 (m, 2H), 4,28-4,31 (d, *J* = 6,0 Hz, 1H), 4,80-4,82 (dd, *J*=2,7/3,9 Hz, 1H), 6,98-7,21 (m, 8H). R_{f} = 0,22 (CHCl₃:AcOEt, 4:1, v/v).

### Step 5. Deprotection of t-Boc group in dipeptidyl derivative of di-4-thiomethylphenyl ester of 1-amino-ethanephosphonic acid.

Dipeptidyl derivative of di-4-thiomethylphenyl ester of 1-amino-ethanephosphonic acid is placed in the flask (0.95 g, 1,68 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,75g which is used without purification in the next step for the synthesis of tripeptidyl derivative of di-4-thiomethylphenyl ester of 1-amino-ethanephosphonic acid.

### Step 6. Synthesis of tripeptidyl derivative of di-4-thiomethylphenyl ester of 1-amino-ethanephosphonic acid.

Into the product obtained in Step 5 - TFA*Pro-Ala^{P}(OC₆H₄-4-S-CH₃)₂ (0,93g; 1,66mmol) 0,37g (1,66mmol) of Boc-Val-OH is added. After 2 minutes from complete dissolving in acetonitrile (15ml), 0,55ml of triethylamine (4mmol) is added. After 10 minutes 0,68g (1.8mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0.82g (74%). Empirical Formula: C₃₁H₄₄N₃O₇PS₂. Molecular weight: 665,80g/mol. ³¹P NMR (CDCl₃, δ [ppm]): δ 19,30 (s, 55%), 19,74 (s, 45%). ¹H NMR (CDCl₃, δ[ppm]) : δ 0,81-1,12 (m, 10H), 1,43 (s, 9H), 1,50-1,57 (m, 2H), 1,75-2,05 (m, 3H), 2,44 (s, 6H), 3,45-3,79 (m, 2H), 4,19-4,32 (m, 1H), 4,33-4,52 (m, 1H), 4,71-4,88 (m, 1H), 5,10-5,27 (m, 1H), 6,98-7,21 (m, 8H). R_{f} = 0,18 (CHCl₃:AcOEt, 4:1, v/v).

### Step 7. Deprotection of t-Boc group in tripeptidyl derivative of di-4-thiomethylphenyl ester of 1-amino-ethanephosphonic acid.

Tripeptidyl derivative of di-4-thiomethylphenyl ester of 1-amino-ethanephosphonic acid is placed in the flask (0.96 g, 1,44 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,81g which is used without purification in the next step for the synthesis of biotinylated tripeptidyl derivative of di-4-thiomethylphenyl ester of 1-amino-ethanephosphonic acid.

### Step 8. Synthesis of biotinylated tripeptidyl derivative of di-4-thiomethylphenyl ester of 1-amino-ethanephosphonic acid.

Product obtained in step 7 (TFA*Val-Pro-Ala^{P}(OC₆H₄-4-S-CH₃)₂ (0,90g, 1,36mmol) is dissolved in N-methylpyrrolidone (10ml) followed by the addition of biotin (0,33g, 1,36mmol). Next, N,N'-diisopropylamine (DIEA) is added in the amount of 0,48ml (3,44mmol). After complete dissolving 0,57g (1,50mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,42g, (39%). Empirical Formula: C₃₆H₅₀N₅O₇PS₃. Molecular weight: 791,98 g/mol. Retention time (HPLC, Alltech Econosphere C-18 10U column, 250×22mm, gradient 5%-95% MeCN/45min): 19,62 min. HRMS *m*/*z* 814,2508/814,2501 (M+Na)⁺.

### Example 5

The method for preparation of bis(4-methoxyphenyl) (3-methyl-1-(1-(3-methyl-2-(5-(2-oxohexahydro-1H-thieno[3,4-d]imidazol-4-yl)pentanamido)butanoyl)pyrrolidine-2-carboxamido)butyl)phosphonate; [Bt-Val-Pro-Leu^{P}(O-C₆H₄-4-O-CH₃)₂] represented by the formula 5.

### Step 1. Synthesis of tri-(4-methoxy)phenyl phosphite.

In the round-bottom flask equipped with a magnetic stirrer 3,72g (30mmol) of 4-methoxyphenol is dissolved in acetonitrile (20ml) followed by the addition of phosphorus (III) chloride (0,87ml, 10mmol). The reaction mixture is then refluxed for 6h. The volatile elements of the mixture are removed under reduced pressure and resulting product obtained as pale yellow oil is used without purification for the synthesis of di-4-methoxyphenyl ester of *N*-(benzyloxycarbonyl-1-amino)-3-methylbutanephosphonic phosphonic acid.

### Step 2. Synthesis of di-4-methoxyphenyl ester of N-(benzyloxycarbonyl-1-amino)-3-methylbutane phosphonic acid.

Into the flask containing tri-(4-methoxy)phenyl phosphite obtained in step 1, 1,51g (10mmol) of benzyl carbamate and 1,30ml (12mmol) of *iso*-valeraldehyde are added followed by the addition of glacial acetic acid (10 ml). The reaction mixture is heated at 90°C for 3h and the volatile components of the mixture are removed under reduced pressure. The product obtained as yellow oil is dissolved in 100-150ml of methanol and stored at -20°C for crystallization (24h). Crystallized product is collected by suction and air-dried obtaining 2,41g (47% yield). Empirical Formula: C₂₇H₃₂NO₇P. Molecular weight: 513,52 g/mol. ³¹P NMR (121MHz, CDCl₃, δ[ppm]): δ 19,51 (s); ¹H NMR (300 MHz, CDCl₃, ppm): δ 7.43-6.70 (m, 13H), 5.25-5.06 (m, 3H), 4.63-4.45 (m, 1H), 3.77 (s, 3H), 3.75 (s, 3H), 1.90-1.67 (m, 3H), 0.97 (d, J=5.7 Hz). mp: 116-118°C. MS *m*/*z* 514,19/514,20 (M+H)⁺

### Step 3. Synthesis of di-4-methoxyphenyl ester of 1-amino-3-methylbutane phosphonic acid hydrobromide salt.

Into the round-bottom flask di-4-methoxyphenyl ester of N-(benzyloxycarbonyl-1-amino)-3-methylbutane phosphonic acid (2,34g, 4,55 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,84 g (88% yield) of di-4-methoxyphenyl ester of 1-amino-3-methylbutane phosphonic acid hydrobromide salt. Empirical Formula: C₁₉H₂₇BrNO₅P. Molecular weight: 460,30g/mol. ³¹P NMR (121MHz, DMSO-*d₆*, δ[ppm]) :δ 13,70 (s); ¹H NMR (300 MHz, DMSO-*d₆*, ppm): 0,93-0,97 (m, 6H), 1,92-2,20 (m, 3H), 3,66 (d, *J =* 3.5 Hz, 6H), 4.01 (t, *J =* 5.6 Hz, 1H), 6,62-7,37 (m, 8H), 8,97 (s, 3H). mp: 159°C.

### Step 4. Synthesis of dipeptidyl derivative of di-4-methoxyphenyl ester of 1-amino-3-methylbutane phosphonic acid.

Into the round-bottom flask 0,92g (2 mmol) of di-4-methoxyphenyl ester of 1-amino-3-methylbutane phosphonic acid hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4: 1) as the eluent. The product is obtained as colorless oil, 1.04g (90%). Empirical Formula: C₂₉H₄₁O₈N₂P. Molecular weight: 576,62 g/mol. ³¹P NMR (121MHz, CDCl₃, δ[ppm]) :δ 19,00 (s); ¹H NMR (300 MHz, CDCl₃, ppm): δ 0,85-0,90 (m, 6H), 1,37 (s, 9H), 1,65-1,90 (m, 7H), 3,50-3,55 (m, 2H), 3,60 (d, *J* = 3.5 Hz, 6H), 4,30-4,34 (m, 1H), 5,20-5,25 (m, 1H), 6,72-7,19 (m, 8H). mp: 96°C.

### Step 5. Deprotection of t-Boc group in dipeptidyl derivative of di-4-methoxyphenyl ester of 1-amino-3-methylbutane phosphonic acid.

Dipeptidyl derivative of di-4-methoxyphenyl ester of 1-amino-3-methylbutane phosphonic acid is placed in the flask (0.97 g, 1,68 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,84g which is used without purification in the next step for the synthesis of tripeptidyl derivative of di-4-methoxyphenyl ester of 1-amino-3-methylbutane phosphonic acid.

### Step 6. Synthesis of tripeptidyl derivative of di-4-methoxyphenyl ester of 1-amino-3-methylbutane phosphonic acid.

Into the product obtained in Step 5 - TFA*Pro-Leu^{P}(OC₆H₄-4-O-CH₃)₂ (0,95g; 1,66mmol) 0,37g (1,66mmol) of Boc-Val-OH is added. After 2 minutes from complete dissolving in acetonitrile (15ml), 0,55ml of triethylamine (4,15 mmol) is added. After 10 minutes 0,68g (1.8mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0.84g (75%). Empirical Formula: C₃₄H₅₀N₃O₉P. Molecular weight: 675,75 g/mol. ³¹P NMR (CDCl₃, δ [ppm]): δ 19,83 (s, 55%), 19,55 (s, 45%). ¹H NMR (CDCl₃, δ[ppm]) :δ 0,92-0,98 (m, 12H), 1,36 (s, 9H), 1,60-2,10 (m, 8H), 3,62-3,68 (m, 2H), 3,69 (s, 6H), 4,22-4,25 (m, 1H), 4,44-4,47 (m, 1H), 4,59-4,81 (m, 1H), 5,28 (d, *J*= 9,3 Hz, 1H), 6,70-7,19 (m, 4H). mp: 62°C. HRMS m/z 698,3182/698,3039 (M+Na)⁺.

### Step 7. Deprotection of t-Boc group in tripeptidyl derivative of di-4-methoxyphenyl ester of 1-amino-3-methylbutane phosphonic acid.

Tripeptidyl derivative of di-4-methoxyphenyl ester of 1-amino-3-methylbutane phosphonic acid is placed in the flask (1.00 g, 1,48 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,92 g which is used without purification in the next step for the synthesis of biotinylated tripeptidyl derivative of di-4-methoxyphenyl ester of 1-amino-3-methylbutane phosphonic acid.

### Step 8. Synthesis of biotinylated tripeptidyl derivative of di-4-thiomethylphenyl ester of 1-amino-ethanephosphonic acid.

Product obtained in step 7 ((TFA*Val-Pro-Leu^{P}(OC₆H₄-4-O-CH₃)₂ (0,92g, 1,36mmol) is dissolved in N-methylpyrrolidone (10ml) followed by the addition of biotin (0,33g, 1,36mmol). Next, N,N'-diisopropylamine (DIEA) is added in the amount of 0,48ml (3,44mmol). After complete dissolving 0,57g (1,50mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,30g, (28%). Empirical Formula: C₃₉H₅₆N₅O₉PS. Molecular weight: 801,93 g/mol. Retention time (HPLC, Alltech Econosphere C-18 10U column, 250x22mm, gradient 5%-95% MeCN/45min): 26,22 min. HRMS m/z 824,3434/824,1826 (M+Na)⁺.

### Example 6

The method for preparation of bis(4-(methylthio)phenyl) (1-(1-(3-methyl-2-(5-(2-oxohexahydro-1H-thieno[3,4-d]imidazol-4-yl)pentanamido)butanoyl)pyrrolidine-2-carboxamido)-2-phenylethyl)phosphonate; [Bt-Val-Pro-Phe^{P}(O-C₆H₄-S-CH₃)₂,] represented by the formula 6.

### Step 1. Synthesis of tri-(4-thiomethyl)phenyl phosphite.

In the round-bottom flask equipped with a magnetic stirrer 4,2g (30mmol) of 4-thiomethylphenol is dissolved in acetonitrile (20ml) followed by the addition of phosphorus (III) chloride (0,87ml, 10mmol). The reaction mixture is then refluxed for 6h. The volatile elements of the mixture are removed under reduced pressure and resulting product obtained as pale yellow oil is used without purification for the synthesis of di-4-thiomethylphenyl ester of N-(benzyloxycarbonyl-1-amino))-2-phenylethanephosphonic acid.

### Step 2. Synthesis of di-4-thiomethylphenyl ester of N-(benzyloxycarbonyl-1-amino)-2-phenylethanephosphonic acid.

Into the flask containing tri-(4-thiomethyl)phenyl phosphite obtained in step 1, 1,51g (10mmol) of benzyl carbamate and 1,45ml (12mmol) of phenylacetaldehyde are added followed by the addition of glacial acetic acid (10 ml). The reaction mixture is heated at 90°C for 3h and the volatile components of the mixture are removed under reduced pressure. The product obtained as yellow oil is dissolved in 100-150ml of methanol and stored at -20°C for crystallization (24h). Crystallized product is collected by suction and air-dried obtaining 2.37g (41% yield). Empirical Formula: C₃₀H₃₀NO₅PS₂. Molecular weight: 579,67 g/mol. ³¹P NMR (121MHz, CDCl₃, δ[ppm]) :δ 18,98 (s); ¹H NMR (300 MHz, CDCl₃, ppm): δ 2,40 (d, *J* = 6.5 Hz, 6H), 2,90-3,36 (m, 2H), 4,70-4,79 (m, 1H), 4,93 (s, 2H), 5,07 (d, *J =* 10.9 Hz, 1H), 6,69-7,28 (m, 18H). mp: 97-99°C.

### Step 3. Synthesis of di-4-thiomethylphenyl ester of 1-amino-2-phenylethanephosphonic acid hydrobromide salt.

Into the round-bottom flask *di*-4-thiomethylphenyl ester of N-(benzyloxycarbonyl-1-amino)-2-phenylethanephosphonic acid (2,00g, 3,45 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,63 g (90% yield) of di-4-thiomethylphenyl ester of 1-amino-ethanephosphonic acid hydrobromide salt. Empirical Formula: C₂₂H₂₅BrNO₃PS₂. Molecular weight: 526,45 g/mol. ³¹P NMR (121MHz, DMSO-*d₆*, δ[ppm]) :δ 15.55 (s); ¹H NMR (300 MHz, DMSO-*d₆*, ppm): δ 2,35 (d, *J* = 7,4 Hz, 6H), 3,23-3,28 (m, 2H), 4,44-4,48 (m, 1H), 6,90-7,36 (m, 13H), 8,76 (s, 3H).

### Step 4. Synthesis of dipeptidyl derivative of di-4-thiomethylphenyl ester of 1-amino-2-phenylethanephosphonic.

Into the round-bottom flask 1,05g (2 mmol) of di-4-thiomethylphenyl ester of 1-amino-2-phenylethanephosphonic hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4: 1) as the eluent. The product is obtained as colorless oil, 0.91g (71%). Empirical Formula: C₃₂H₃₉O₆N₂PS₂. Molecular weight: 642,77 g/mol. ³¹P NMR (121MHz, CDCl₃, δ[ppm]): δ 18,24 (s); ¹H NMR (300 MHz, CDCl₃, ppm): δ 1.40 (s, 9H), 1,65-1,90 (m, 4H), 2,35 (s, 6H), 3,40-3,54 (m, 4H), 4,12-4,15 (m, 1H), 5,00-5,10 (m, 1H), 5,19 (s, 1H), 6,94-7,18 (m, 13H). mp: 49-52°C.

### Step 5. Deprotection of t-Boc group in dipeptidyl derivative of di-4-thiomethylphenyl ester of 1-amino-2-phenylethanephosphonic acid.

Dipeptidyl derivative of di-4-thiomethylphenyl ester of 1-amino-2-phenylethanephosphonic acid is placed in the flask (1.08 g, 1,68 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,92 g which is used without purification in the next step for the synthesis of tripeptidyl derivative of di-4-thiomethylphenyl ester of 1-amino-2-phenylethanephosphonic acid.

### Step 6. Synthesis of tripeptidyl derivative of di-4-thiomethylphenyl ester of 1-amino-2-phenylethanephosphonic acid.

Into the product obtained in Step 5 - TFA*Pro-Phe^{P}(OC₆H₄-4-S-CH₃)₂ (1,06g; 1,66mmol) 0,37g (1,66mmol) of Boc-Val-OH is added. After 2 minutes from complete dissolving in acetonitrile (15ml), 0,55ml of triethylamine (4mmol) is added. After 10 minutes 0,68g (1.8mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0.93g (76%). Empirical Formula: C₃₇H₄₈N₃O₇PS₂. Molecular weight: 741,90 g/mol. ³¹P NMR (CDCl₃, δ [ppm]): δ 18,30 (s, 55%), 18.26 (s, 45%). ¹H NMR (CDCl₃, δ[ppm]): δ 0,88-0,92 (m, 6H), 1,37 (s, 9H), 1,65-1,80 (m, 5H), 2,35-2,40 (m, 6H), 3,15-3,17 (m, 2H), 3,50-3,55 (m, 2H), 4,99 (s, 1H), 4,16-4,18 (m, 1H), 5,11-5,13 (m, 1H), 5,16 (s, 1H), 6,79-7,01 (m, 8H). HRMS m/z 764,2569/764.2250 (M+Na)⁺.

### Step 7. Deprotection of t-Boc group in tripeptidyl derivative of di-4-thiomethylphenyl ester of 1-amino-2-phenylethanephosphonic acid.

Tripeptidyl derivative of di-4-thiomethylphenyl ester of 1-amino-2-phenylethanephosphonic acid is placed in the flask (1.1 g, 1,48 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,83g which is used without purification in the next step for the synthesis of biotinylated tripeptidyl derivative of di-4-thiomethylphenyl ester of 1-amino-2-phenylethanephosphonic acid.

### Step 8. Synthesis of biotinylated tripeptidyl derivative of di-4-thiomethylphenyl ester of 1-amino-ethanephosphonic acid.

Product obtained in step 7 (TFA*Val-Pro-Phe^{P}(OC₆H₄-4-S-CH₃)₂ (1,00 g, 1,36mmol) is dissolved in N-methylpyrrolidone (10ml) followed by the addition of biotin (0,33g, 1,36mmol). Next, N,N'-diisopropylamine (DIEA) is added in the amount of 0,48ml (3,44mmol). After complete dissolving 0,57g (1,50mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,42g, (36%). Empirical Formula: C₄₂H₅₄N₅O₇PS₃. Molecular weight: 868,08 g/mol. Retention time (HPLC, Alltech Econosphere C-18 10U column, 250x22mm, gradient 5%-95% MeCN/45min): 22,58 min. HRMS *m*/*z* 890,2820/890,2310 (M+Na)⁺.

### Example 7

The method for preparation of diphenyl (3-methyl-1-(1-(3-methyl-2-(5-(2-oxohexahydro-1H-thieno[3,4-d]imidazol-4-yl)pentanamido)butanoyl)pyrrolidine-2-carboxamido)butyl)phosphonate [Bt-Val-Pro-Leu^{P}(O-C₆H₅)₂] represented by the formula 7.

### Step 1. Synthesis of diphenyl ester of N-(benzyloxycarbonyl-1-amino)-3-methylbutanephosphonic acid.

Into the round-bottom flask containing triphenyl phosphite (3,10g, 10mmol) benzyl carbamate, (1,51g, 10mmol) and isovaleraldehyde 1,1ml (10,5 mmol) are added followed by the addition of glacial acetic acid (10 ml). The reaction was performed at 90°C for 4h. The volatile elements of the reaction mixture were removed in vacuum and the resulting oil was dissolved in methanol (100-150ml). The solution is placed at -20°C for 24 h to complete crystallization. The product is collected by suction and air-dried to obtain 2,45g (54% yield). Empirical Formula: C₂₅H₂₈NO₅P. Molecular weight: 453,17 g/mol. ³¹P NMR (121MHz, CDCl₃, δ[ppm]): δ 19,82 (s); ¹H NMR (300 MHz, CDCl₃, ppm): δ 0,91 (d, J=5,6 Hz, 6H), 1,62-1,81 (m, 3H), 4,45-4,58 (m, 1H), 4,93 (d, J=10,4 Hz, 1H), 5,02 (d, J=12,2 Hz, 2H), 5,09 (d, J=12,2 Hz, 2H), 7,01-7,27 (m, 15H).

### Step 2. Synthesis of diphenyl ester of 1-amino-3-methylbutanephosphonic acid hydrobromide salt.

Into the round-bottom flask diphenyl ester of N-(benzyloxycarbonyl-1-amino)-3-methylbutanephosphonic acid (2,00g, 4.41 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,46 g (83% yield) of diphenyl ester of 1-amino-3-methylbutanephosphonic acid hydrobromide salt. Empirical Formula: C₁₇H₂₃BrNO₃P. Molecular weight: 399,06g/mol. ³¹P NMR (121 MHz, DMSO-d₆, δ[ppm]): δ 15,61 (s); ¹H NMR (300 MHz, DMSO-d₆, ppm): δ 0,90-0,94 (m, 6H), 1,79-1,84 (m, 3H), 4,32-4,35 (m, 1H), 7,37-8,02 (m, 10H), 8,73 (s, 3H).

### Step 3. Synthesis of dipeptidyl derivative of diphenyl ester of 1-amino-3-methylbutanephosphonic acid.

Into the round-bottom flask 0,80g (2 mmol) of diphenyl ester of 1-amino-3-methylbutanephosphonic acid hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4: 1) as the eluent. The product is obtained as colorless oil, 0,93 g (90%). Empirical Formula: C₂₇H₃₇O₆N₂P. Molecular weight: 516,24 g/mol. ³¹P NMR (121MHz, CDCl₃, δ[ppm]): δ 18,78 (s, 49%), 18,79 (s, 51%). ¹H NMR (300 MHz, CDCl₃, ppm): δ 0,89 (d, *J* = 3.6, 6H), 1,36 (s, 9H), 1,36-1,79 (m, 6H), 2,73-2,78 (m, 3H), 3,27-3,33 (m, 1H), 4,24-4,26 (m, 1H), 4,87 (d, *J* = 5.9 Hz, 1H), 6,97-7,28 (m, 10H).

### Step 4. Deprotection of t-Boc group in dipeptidyl derivative of diphenyl ester of 1-amino-3-methylbutanephosphonic acid.

Dipeptidyl derivative of diphenyl ester of diphenyl ester of 1-amino-3-methylbutanephosphonic acid is placed in the flask (0.83 g, 1.6 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil (0.81g) which is used without purification in the next step for the synthesis of tripeptidyl derivative of diphenyl ester of 1-amino-3-methylbutanephosphonic acid.

### Step 5. Synthesis of tripeptidyl derivative of diphenyl ester of 1-amino-3-methylbutanephosphonic acid.

Into the product obtained in Step 4 - TFA*Pro-Leu^{P}(OC₆H₅)₂ (0,81 g; 1,51 mmol) 0,33g (1,51mmol) of Boc-Val-OH is added. After 2 minutes from complete dissolving in acetonitrile (15ml), 0,52ml of triethylamine (3,76mmol) is added. After 10 minutes 0,63g (1.66mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0.86g (93%). Empirical Formula: C₃₂H₄₆N₃O₇P. Molecular weight: 615,31 g/mol. ³¹P NMR (CDCl₃, δ [ppm]): δ 18.63 (s, 40%), 18.84 (s, 60%). ¹H NMR (CDCl₃, δ[ppm]): 0,83-0,97 (m, 12H), 1,26-1,28 (m, 2H), 1,44 (s, 9H), 1,80-1,85 (m, 1H), 1,90-2,00 (m, 2H), 2,10-2,20 (m, 2H), 3,69-3,73 (m, 2H), 4,18-4,20 (m, 1H), 4,40-4,56 (m, 1H), 4,85-4,92 (m, 1H), 5,29 (s, 1H), 6,81-7,42 (m, 10H). HRMS *m*/*z* 638.2971/638.2974 (M+Na)⁺.

### Step 6. Deprotection of t-Boc group in tripeptidyl derivative of diphenyl ester of 1-amino-3-methylbutanephosphonic acid.

Tripeptidyl derivative of diphenyl ester of 1-amino-3-methylbutanephosphonic acid is placed in the flask (0.80 g, 1,30 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil (0,79g) which is used without purification in the next step for the synthesis of biotinylated tripeptidyl derivative of diphenyl ester of 1-amino-3-methylbutanephosphonic acid.

### Step 7. Synthesis of biotinylated tripeptidyl derivative of diphenyl ester of 1-amino-3-methylbutanephosphonic acid.

Product obtained in step 6 TFA*Val-Pro-Leu^{P}(OC₆H₅)₂ (0,79g, 1,26mmol) is dissolved in N-methylpyrrolidone (10ml) followed by the addition of biotin (0,31g, 1,26mmol). Next, N,N'-diisopropylamine (DIEA) is added in the amount of 0,54ml (3,15mmol). After complete dissolving 0,53g (1,39mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,29g, (29%). Empirical Formula: C₃₇H₅₂N₅O₇PS. Molecular weight: 741,33 g/mol. Retention time (HPLC, Alltech Econosphere C-18 10U column, 250x22mm, gradient 5%-95% MeCN/45min): 19,80 min. HRMS *m*/*z* 764,3223/764,3000 (M+Na)⁺.

### Example 8

The method for preparation of bis(4-(methylthio)phenyl) (3-methyl-1-(1-(5-(2-oxohexahydro-1H-thieno[3,4-d]imidazol-4-yl)pentanoyl)pyrrolidine-2-carboxamido)butyl)phosphonate [Bt-Pro-Leu^{P}(O-C₆H₄-4-S-CH₃)₂] represented by the formula 8.

### Step 1. Synthesis of tri-(4-thiomethyl)phenyl phosphite.

In the round-bottom flask equipped with a magnetic stirrer 4,2g (30mmol) of 4-thiomethylphenol is dissolved in acetonitrile (20ml) followed by the addition of phosphorus (III) chloride (0,87ml, 10mmol). The reaction mixture is then refluxed for 6h. The volatile elements of the mixture are removed under reduced pressure and resulting product obtained as pale yellow oil is used without purification for the synthesis of di-4-thiomethylphenyl ester of *N*-(benzyloxycarbonyl-1-amino)-3-methylbutane phosphonic acid.

### Step 2. Synthesis of di-4-thiomethylphenyl ester of N-(benzyloxycarbonyl-1-amino)-3-methylbutane phosphonic acid.

Into the flask containing tri-(4-thiomethyl)phenyl phosphite obtained in step 1, 1,51g (10mmol) of benzyl carbamate and 1,3ml (12mmol) of *iso*valeraldehyde are added followed by the addition of glacial acetic acid (10 ml). The reaction mixture is heated at 90°C for 3h and the volatile components of the mixture are removed under reduced pressure. The product obtained as yellow oil is dissolved in 100-150ml of methanol and stored at -20°C for crystallization (24h). Crystallized product is collected by suction and air-dried obtaining 2.35g (43% yield). Empirical Formula: C₂₇H₃₂NO₅PS₂. Molecular weight: 545,65 g/mol. ³¹P NMR (121MHz, CDCl₃, δ[ppm]): δ 20,37 (s); ¹H NMR (300 MHz, CDCl₃, ppm): δ 0,98 (d, J = 5,8 Hz, 6H), 1,61-2,16 (m, 3H), 2,47 (s, 6H), 4,61-4,63 (m, 1H), 5,06-5,17 (m, 2H), 5,18 (s, 1H), 6,90-7,30 (m, 13H).

### Step 3. Synthesis of di-4-thiomethylphenyl ester of 1-amino-3-methylbutane phosphonic acid hydrobromide salt.

Into the round-bottom flask di-4-thiomethylphenyl ester of N-(benzyloxycarbonyl-1-amino)-3-methylbutane phosphonic acid (2,00g, 3,67 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,63 g (90% yield) of di-4-thiomethylphenyl ester of 1-amino-3-methylbutane phosphonic acid hydrobromide salt. Empirical Formula: C₁₉H₂₇BrNO₃PS₂. Molecular weight: 492,43 g/mol. ³¹P NMR (121MHz, DMSO-*d*₆, δ[ppm]): δ 15,55 (s); ¹H NMR (300 MHz, DMSO-*d*₆, ppm): δ 0,92-1,11 (m, 6H), 1,15-2,01 (m, 3H), 2,35 (s, 6H), 4,44-4,46 (m, 1H), 6,90-7,36 (m 8H), 8,76 (s, 3H).

### Step 4. Synthesis of dipeptidyl derivative of di-4-thiomethylphenyl ester of 1-amino-3-methylbutane phosphonic acid.

Into the round-bottom flask 0,98g (2 mmol) of di-4-thiomethylphenyl ester of 1-amino-3-methylbutane phosphonic acid hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4: 1) as the eluent. The product is obtained as colorless oil, 1.12g (92%). Empirical Formula: C₂₉H₄₁O₆N₂PS₂. Molecular weight: 608,75 g/mol. ³¹P NMR (121MHz, CDCl₃, δ[ppm]): δ 19,00 (s, 43%), 18,83 (s, 57%); ¹H NMR (300 MHz, CDCl₃, ppm): δ 0,85-0,87 (m, 6H), 1,37 (s, 9H), 1,65-1,90 (m, 7H), 3,50-3,52 (m, 2H), 3,51 (s, 6H), 4,30-4,35 (m, 1H), 5,20-5,21 (m, 1H), 6,72-7,19 (m, 8H).

### Step 5. Deprotection of t-Boc group in dipeptidyl derivative of di-4-thiomethylphenyl ester of 1-amino-3-methylbutane phosphonic acid.

Dipeptidyl derivative of di-4-thiomethylphenyl ester of 1-amino-3-methylbutane phosphonic acid. is placed in the flask (1.0 g, 1,64 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,99 g which is used without purification in the next step for the synthesis of biotinylated dipeptidyl derivative of di-4-thiomethylphenyl ester of 1-amino-3-methylbutane phosphonic acid.

### Step 6. Synthesis of biotinylated dipeptidyl derivative of di-4-thiomethylphenyl ester of 1-amino-3-methylbutane phosphonic acid.

Product obtained in step 7 TFA* Pro-Leu^{P}(OC₆H₄-4-S-CH₃)₂ (0,96g, 1,33mmol) is dissolved in N-methylpyrrolidone (10ml) followed by the addition of biotin (0,32g, 1,33mmol). Next, N,N'-diisopropylamine (DIEA) is added in the amount of 0,58ml (3,33mmol). After complete dissolving 0,55g (1,46mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,21g, (21%). Empirical Formula: C₃₄H₄₇N₄O₆PS₃. Molecular weight: 734,24 g/mol. Retention time (HPLC, Alltech Econosphere C-18 10U column, 250x22mm, gradient 5%-95% MeCN/45min): 21,13 min. HRMS *m*/*z* 757,2293-757,2303 (M+Na)⁺.

### Example 9

The method for preparation of dimethyl 4,4'-(((3-methyl-1-(1-(3-methyl-2-(5-(2-oxohexahydro-1H-thieno[3,4-d]imidazol-4-yl)pentanamido)butanoyl)pyrrolidine-2-carboxamido)butyl)phosphoryl)bis(oxy))dibenzoate [Bt-Val-Pro-Leu^{P}(O-C₆H₄-4-COOCH₃)₂] represented by the formula 9.

### Step 1. Synthesis of tri-(4-carboxymethyl)phenyl phosphite.

In the round-bottom flask equipped with a magnetic stirrer 4,56g (30mmol) of 4-carboxymethyl phenol is dissolved in acetonitrile (20ml) followed by the addition of phosphorus (III) chloride (0,87ml, 10mmol). The reaction mixture is then refluxed for 6h. The volatile elements of the mixture are removed under reduced pressure and resulting product obtained as pale yellow oil is used without purification for the synthesis of di-4-carboxymethylphenyl ester of *N-*(benzyloxycarbonyl-1-amino)-3-methylbutanephosphonic phosphonic acid.

### Step 2. Synthesis of di-4-carboxymethylphenyl ester of N-(benzyloxycarbonyl-1-amino)-3-methylbutanephosphonic phosphonic acid.

Into the flask containing tri-(4-carboxymethyl)phenyl phosphite obtained in step 1, 1,51g (10mmol) of benzyl carbamate and 1,30ml (12mmol) of isovaleraldehyde are added followed by the addition of glacial acetic acid (10 ml). The reaction mixture is heated at 90°C for 3h and the volatile components of the mixture are removed under reduced pressure. The product obtained as yellow oil is dissolved in 100-150ml of methanol and stored at -20°C for crystallization (24h). Crystallized product is collected by suction and air-dried obtaining 2,45g (43% yield). Empirical Formula: C₂₉H₃₂NO₉P. Molecular weight: 569,54 g/mol. ³¹P NMR (121MHz, CDCl₃, δ[ppm]): δ 19.53 (s); ¹H NMR (300 MHz, CDCl₃, ppm): δ 0,90 (d, *J* = 5.7 Hz, 6H), 1,79-1,82 (m, 3H), 3,82 (s, 6H), 4,52-4,61 (m, 1H), 5,01-5,16 (m, 3H), 6,92-7,93 (m, 13H).mp: 87°C.

### Step 3. Synthesis of di-4-carboxymethylphenyl ester of 1-amino-3-methylbutanephosphonic phosphonic acid hydrobromide salt.

Into the round-bottom flask di-4-carboxymethylphenyl ester of N-(benzyloxycarbonyl-1-amino)-3-methylbutanephosphonic phosphonic acid (2,00g, 3,51 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,60 g (88% yield) of di-4-carboxymethylphenyl ester of 1-amino-3-methylbutanephosphonic phosphonic acid hydrobromide salt. Empirical Formula: C₂₁H₂₇BrNO₇P. Molecular weight: 516,32 g/mol. ³¹P NMR (121MHz, DMSO-d₆, ppm): δ 16,42 (s); ¹H NMR (300 MHz, DMSO-d₆, ppm): δ 0,91-0,96 (m, 6H), 1,81-1,85 (m, 3H), 3,80 (s, 6H), 4,30-4,33 (m, 1H), 7,36-8,03 (m, 8H), 8,77 (s, 3H).

### Step 4. Synthesis of dipeptidyl derivative of di-4-carboxymethylphenyl ester of 1-amino-3-methylbutanephosphonic phosphonic acid.

Into the round-bottom flask 1,03g (2 mmol) of di-4-carboxymethylphenyl ester of *1-amino-3-methylbutanephosphonic* phosphonic acid hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0,78 g (62%). Empirical Formula: C₃₁H₄₁O₁₀N₂P. Molecular weight: 632,64 g/mol. ³¹P NMR (121MHz, CDCl₃, ppm): δ 19,62 (s); ¹H NMR (300 MHz, CDCl₃, ppm): δ 0,81-0,85 (m, 6H), 1,31 (s, 9H), 1,66-2,21 (m, 7H), 3,26-3,30 (m, 2H), 3,76 (s, 6H), 4,17-4,19 (m, 1H), 4,78-4,80 (m, 1H), 7,27-7,75 (m, 8H).

### Step 5. Deprotection of t-Boc group in dipeptidyl derivative of di-4-carboxymethylphenyl ester of 1-amino-3-methylbutanephosphonic phosphonic acid.

Dipeptidyl derivative of di-4-carboxymethylphenyl ester of 1-amino-3-methylbutanephosphonic phosphonic acid is placed in the flask (1.06 g, 1,68 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,86g which is used without purification in the next step for the synthesis of tripeptidyl derivative of di-4-carboxymethylphenyl ester of 1-amino-3-methylbutanephosphonic phosphonic acid.

### Step 6. Synthesis of tripeptidyl derivative of di-4-carboxymethylphenyl ester of 1-amino-3-methylbutanephosphonic phosphonic acid.

Into the product obtained in Step 5 - TFA*Pro-Leu^{P}(OC₆H₄-4-COOCH₃)₂ (1,05 g; 1,66 mmol) 0,37g (1,66mmol) of Boc-Val-OH is added. After 2 minutes from complete dissolving in acetonitrile (15ml), 0,55ml of triethylamine (4,15 mmol) is added. After 10 minutes 0,68g (1.8mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0.84g (69%). Empirical Formula: C₃₆H₅₀N₃O₁₁P. Molecular weight: 731,77 g/mol. ³¹P NMR (CDCl₃, ppm): δ 19,83 (s, 43%), 19,55 (s, 57%). ¹H NMR (CDCl₃, ppm): 80,92-0,95 (m, 12H), 1,36 (s, 9H), 1,60-2,10 (m, 8H), 3,62-3,68 (m, 2H), 3,79 (s, 6H), 4,22-4,25 (m, 1H), 4,44-4,48 (m, 1H), 4,59-5,00 (m, 1H), 5,28 (d, *J* = 9.3 Hz), 6,70-7,19 (m, 8H).

### Step 7. Deprotection of t-Boc group in tripeptidyl derivative of di-4-carboxymethylphenyl ester of 1-amino-3-methylbutanephosphonic phosphonic acid.

Tripeptidyl derivative of di-4-carboxymethylphenyl ester of 1-amino-3-methylbutanephosphonic phosphonic acid is placed in the flask (1.05 g, 1,44 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,93 g which is used without purification in the next step for the synthesis of biotinylated tripeptidyl derivative of di-4-carboxymethylphenyl ester of 1-amino-3-methylbutanephosphonic phosphonic acid.

### Step 8. Synthesis of biotinylated tripeptidyl derivative of di-4-carboxymethylphenyl ester of 1-amino-3-methylbutanephosphonic phosphonic acid.

Product obtained in step 7 (TFA*Val-Pro-Leu^{P}(OC₆H₄-4-COOCH₃)₂ (0,99 g, 1,36 mmol) is dissolved in N-methylpyrrolidone (10ml) followed by the addition of biotin (0,33g, 1,36mmol). Next, N,N'-diisopropylamine (DIEA) is added in the amount of 0,48ml (3,44mmol). After complete dissolving 0,57g (1,50mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,54g, (46%). Empirical Formula: C₃₆H₅₀N₅O₇PS₃. Molecular weight: 791,98 g/mol. Retention time (HPLC, Alltech Econosphere C-18 10U column, 250x22mm, gradient 5%-95% MeCN/45min): 26,49 min. HRMS *m*/*z* 880,3359/880,3408 (M+Na)⁺.

### Example 10

The method for preparation of diphenyl (1-(1-(3-methyl-2-(4-oxo-4-((5-(5-(2-oxohexahydro-1H-thieno[3,4-d]imidazol-4-yl)pentanamido)pentyl)amino)butanamido)butanoyl)pyrrolidine-2-carboxamido)-2-phenylethyl)phosphonate [Bt-LC-Suc-Val-Pro-Phe^{P}(O-C₆H₅)₂] represented by the formula 10.

### Steps from 1 to 6 were described in example 3.

### Step 7. Synthesis of succinyl tripeptidyl derivative of diphenyl ester of 1-amino-2-phenylethane phosphonic acid.

Into the product obtained in Step 6 - (TFA*Val-Pro-Phe^{P}(OC₆H₅)₂ (0,88 g, 1,36 mmol) is dissolved in ethyl acetate (50ml) followed by the addition of succinic anhydride (1,5g, 1,50mmol). Next, N,N'-diisopropylamine (DIEA) is added in the amount of 0,48ml (3,44mmol) and the reaction is performed at room temperature for 24h. Next 100 ml of ethyl acetate is added to the reaction mixture and the solution is washed with KHSO₄aq (5%), brine, NaHCO₃aq (5%), and brine and dried over magnesium sulfate for 2h. Next, volatile elements are removed in vacuum and the product is obtained as colorless oil, 0,80 g (91%). Empirical Formula: C₃₄H₄₀N₃O₈P. Molecular weight: 649,67 g/mol. ³¹P NMR (CDCl₃, ppm): δ 18,53 (s, 50%), 17,48 (s, 50%). ¹H NMR (CDCl₃, ppm): δ 0,86-0,94 (m, 6H), 1,11-1,39 (m, 2H), 1,40-1,45 (m, 1H), 1,51-2,0 (m, 3H), 2,51-2,61 (m, 4H), 2,98-3,06 (m, 1H), 3,38-3,51 (m, 2H), 3,71-3,84 (m, 1H) 4,46-4.50 (m, 1H), 5,13-5,14 (m,1H), 7,03-7,36 (m, 15H), 7,50 (d, *J* = 2,1 Hz, 1H), 7,88 (d, *J* = 9,0 Hz, 1H).

### Step 8. Synthesis of biotinylated succinyl tripeptidyl derivative of diphenyl ester of 1-amino-2-phenylethane phosphonic acid.

Product obtained in step 7 (Suc-Val-Pro-Phe^{P}(OC₆H₅)₂ (88,5 mg, 0,136mmol) is dissolved in N-methylpyrrolidone (2ml) followed by the addition of biotin pentylamine (Bt-LC-NH₂ (44,5 mg, 0,136mmol). Next, N,N'-diisopropylamine (DIEA) is added in the amount of 48µl (0,344mmol). After complete dissolving 57mg (0,15mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 27,4, (21%). Empirical Formula: C₄₉H₆₆N₇O₉PS. Molecular weight: 960,13 g/mol. Retention time (HPLC, Alltech Econosphere C-18 10U column, 250x22mm, gradient 5%-95% MeCN/45min): 19,80 min. HRMS *m*/*z* 960,4459/960,4481 (M+H)⁺.

### Example 11

The method for preparation of bis(4-(methylthio)phenyl) 10-benzyl-1-(4-guanidinophenyl)-4-(1-hydroxyethyl)-7-isopropyl-3,6,9,12,15,23-hexaoxo-27-(2-oxohexahydro-1H-thieno[3,4-d]imidazol-4-yl)-2,5,8,11,16,22-hexaazaheptacosylphosphonate; [Bt-LC-Suc-Phe-Val-Thr-(4-Gu)Phg^{P}(O-C₆H₄-4-S-CH₃)₂, represented by the formula 11.

### Step 1. Synthesis of t-butyl monoester of succinic acid.

Into the round-bottom flask succinic anhydride (3,00 g, 30 mmol), N-hydroxysuccinimide (1,00 g, 9 mmol) and 4-(dimethylamino)pyridine (0,35 g, 2,86 mmol) are added and dissolved in toluene (50 ml). Next, tert-butyl alcohol (5 ml) and triethylamine (1,25 ml, 9 mmol) are added. The reaxtion is refluxed for 24 h. After cooling ethyl acetate (50 ml) is added into the reaction mixture and washed with citric acid (5%) and brine. Organic fraction is dried over magnesium sulfate and evaporated to dryness. Obtained oil is dissolved in diethyl ether/hexane solution (1/3, v/v) and left for crystallization at room temperature. The product if collected by suction: 3.65 g (70% wagowo). Empirical Formula: C₈H₁₄O₄. Molecular weight: 174,19 g/mol. ¹H NMR (300 MHz, DMSO-d₆, δ [ppm]): δ 1,44 (s, 9H), 2,51-2,68 (m, 4H).

### Step 2. Synthesis of tBu-Suc-Phe-Val-Thr(O-tBu)-OH.

Synthesis of *tBu-Suc-Phe-Val-Thr(O-tBu)-OH* is performed on solid-phase staring with 2-chloro-trityl resin containing attached threonine(O-tBu) residue (1,00 g, 0,92 mmol of threonine). Resin is first washed with dichloromethane and dimethylformamide (DMF) and Fmoc-Val-OH (0,94 g, 2,76 mmol) dissolved in DMF (2 ml) is added. After 5 minutes PyBOP (1,44 g, 2,76 mmol) and diisopropylamine (DIPEA, 1.2ml) are added. The coupling reaction is performer at room temperature for 2h. After washing the reaction mixture with DMF, the Fmoc deprotection is performed using 20% piperidine/DMF solution for 25 minutes. After washing with DMF next coupling steps are performed in similar way using the following reagents: Fmoc-Phe-OH (1,07 g, 2,76 mmol) and t-butyl monoester of succinic acid (0,48 g, 2,76 mmol). After final DMF washing the resin is washed with dichloromethane, methanol and hexane. In order to cleave peptide off the resin 30% trifluoroethanol in dichloromethane is used. The reaction is performed for 2h at room temperature. The filtered peptide solution is concentrated in vacuum leading to desired product obtained as pale yellow oil 0,47 g (88%). Empirical formula: C₃₀H₄₇N₃O₈. Molecular weight: 577,71 g/mol. HRMS m/z 600,3261/600,2151 (M+Na)⁺.

### Step 3. Synthesis of tri-(4-thiomethyl)phenyl phosphite.

In the round-bottom flask equipped with a magnetic stirrer 4,2g (30mmol) of 4-thiomethylphenol is dissolved in acetonitrile (20ml) followed by the addition of phosphorus (III) chloride (0,87ml, 10mmol). The reaction mixture is then refluxed for 6h. The volatile elements of the mixture are removed under reduced pressure and resulting product obtained as pale yellow oil is used without purification for the synthesis of di-4-thiomethylphenyl ester of N-(benzyloxycarbonyl-1-amino)-3-methylbutane phosphonic acid.

### Step 4. Synthesis of di-4-thiomethylphenyl ester of N-(benzyloxycarbonyl-1-amino)-(4-nitrophenyl)methanephosphonic acid.

Into the flask containing tri-(4-thiomethyl)phenyl phosphite obtained in step 1, 1,51g (10mmol) of benzyl carbamate and 1,51g (10mmol) of 4-nitrophenyl aldehyde are added followed by the addition of glacial acetic acid (10 ml). The reaction mixture is heated at 90°C for 3h and the volatile components of the mixture are removed under reduced pressure. The product obtained as yellow oil is dissolved in 100-150ml of methanol and stored at -20°C for crystallization (24h). Crystallized product is collected by suction and air-dried obtaining 3.9g (64% yield). Empirical formula: C₂₉H₂₇N2O₇PS₂. Molecular weight: 610,64 g/mol. ³¹P NMR (121MHz, CDCl₃, δ[ppm]): δ 13,66 (s); ¹H NMR (300 MHz, CDCl₃, ppm): δ 5,01-5,17 (m, 2H), 5,65 (dd, J = 9,0/23,4 Hz, 1H), 6,13 (dd, J = 5,7/8,8 Hz, 1H), 6,91-8,19 (m, 19H). mp: 160-161°C.

### Step 5. Synthesis of di-4-thiomethylphenyl ester of N-(benzyloxycarbonyl-1-amino)-(4-aminophenyl)methanephosphonic acid.

Into the round-bottom flask di-4-thiomethylphenyl ester of N-(benzyloxycarbonyl-1-amino)-(4-nitrophenyl)methanephosphonic acid (1,83 g , 3 mmol) is added and dissolved in ethyl acetate (60 ml) followed by the addition of SnCl₂ (3,30 g, 17,4 mmol) and water (1,08 ml, 60 mmol). The reaction mixture is refluxed for 48 h. After the reaction is completed 250 ml of ethyl acetate is added and sodium hydroxide solution (1N) is added until the pH=7-8. The mixture is filtered through a pad of Celite, dried over magnesium sulfate and evaporated to dryness. The product is obtained as yellow solid: 1,74 g (99%). Empirical formula: C₂₉H₂₉N2O₅PS₂. Molecular weight: 580,65 g/mol. ³¹P NMR (121MHz, CDCl₃, δ[ppm]): δ 17,38 (s); ¹H NMR (300 MHz, CDCl₃, ppm): δ 4,98 (d, J = 12,5 Hz, 1H), 5,06 (d, J = 12,4 Hz, 1H), 5,13 (s, 2H), 5,28 (dd, J = 10,1/20,1 Hz), 6,47-7,30 (m, 19H), 8,61 (d, J = 10,1, 1H). mp: 176-178°C.

### Step 6. Synthesis of di-4-thiomethylphenyl ester of 1-amino-(4-aminophenyl)methanephosphonic acid.

Into the round-bottom flask di-4-thiomethylphenyl ester of N-(benzyloxycarbonyl-1-amino)-(4-aminophenyl)methanephosphonic acid (2,00g, 3,44 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 2.01 g (96% yield). Empirical Formula: C₂₁H₂₅Br₂N₂O₃PS₂. Molecular weight: 608,35 g/mol. ³¹P NMR (121MHz, DMSO-*d*₆, δ[ppm]): δ 12,68 (s); ¹H NMR (300 MHz, DMSO-*d*₆, ppm): δ 2,43 (d, J = 7,5 Hz, 6H), 5,45 (d, J = 12,5 Hz), 6,81-7,43 (m, 12H), 9,21 (s, 4H).

### Etap 7. Synthesis of N,N'-di-Boc-S-ethyloisothiourea.

Into the round-bottom flask thiourea (7.62 g, 0.1 mol) is added and dissolved in absolute ethyl alcohol (50ml) followed by the addition of freshly distilled bromoethane (8.3 ml, 0.11 mol). The reaction mixture is refluxed for 4 h and the volatile components are removed in vacuum. Resulting oil is dissolved in diethyl ether (100 ml) add left for crystallization at room temperature. The white crystalline powder is collected by suction and air-dried. The product is placed in the round-bottom flask (1.85 g, 0.01 mol) and dissolved in dry dichloromethane (25 ml) followed by the addition of di-*tert*-butyl bicarbonate (0.48 g, 0.022 mol) and DIPEA (3.6 ml, 0.022 mol). The reaction is performed at room temperature for 4-5 days and its progres is monitored by TLC. The reaction mixture is washed with brine, 5% NaHCO₃aq, 5% citric acid and water. The organic layer is collected, dried over magnesium sulfate, evaporated to dryness and left for crystallization at room temperature. The product is obtained as white crystals: 2,55 g (84%). Empirical Formula: C₁₃H₂₄N₂O₄S. Molecular weight: 304,41 g/mol. ¹H NMR (300 MHz, CDCl₃, ppm): δ 1,24 (t, J = 4,2 Hz, 3H), 1,48 (d, J = 4.1, 18H), 3,01 (q, J = 7,5 Hz, 2H), 11,62 (s, 1H). mp: 69°C.

### Step 8. Synthesis of tBu-Suc-Phe-Val-Thr-(4-NH₂)Phg^{P}(O-C₆H₄-4-S-CH₃)₂.

Into the round-bottom flask peptide obtained in Step 2 (t-Bu-Suc-Phe-Val-Thr(O-tBu)-OH) is placed (240 mg, 0,41 mmol) and dissolved in acetonitrile (10 ml). Next, hydrobromide salt obtained in Step 6 is added (255 mg, 0,41 mmol). The reaction is cooled in an ice bath and 0,21ml of triethylamine (1,48 mmol) is added. After 10 minutes 190 mg (0,49 mmol) of HBTU is added. The reaction is performed for 24 godziny at room temperature. The volatile components are removed in vacuum and the resulting oil is dissolved in ethyl acetate (50 ml) followed by washing with 5% KHSO₄aq, brine, 5% NaHCO₃aq and brine. The organic layer is dried over magnesium sulfate and evaporated to dryness. The obtained product (360 mg) is used in next step without purificationEmpirical Formula: C₅₁H₆₈O₁₀N₅PS₂. Molecular weight: 1006,22 g/mol. HRMS *m*/*z* 1028,4043/1028,1646 (M+Na)⁺.

### Step 9. Synthesis of tBu-Suc-Phe-Val-Thr-(4-Boc₂Gu)Phg^{P}(O-C₆H₄-4-S-CH₃)₂.

In the round-bottom flask product obtained in Step 8 (340 mg, 0,33 mmol) is dissolved in dry chloroform (30 ml). Next, N,N'-di-Boc-S-ethyloisothiourea (155 mg, 049 mmol), triethzlamine (0,14 ml, 1 mmol) and HgCl₂ (850 mg, 3,9 mmol) are added. The reaction is performed at room temperature for 48h. The reaction mixture is passed through a pad of Celite, volatile components are removed in vacuum, and the resulting oil is dissolved in ethyl acetate (50 ml) followed by washing with 5% KHSO₄aq, brine, 5% NaHCO₃aq and brine. The organic layer is dried over magnesium sulfate and evaporated to dryness. The product (320 mg) is used directly in the next step without purification. Empirical formula: C₆₂H₈₆O₁₄N₇PS₂. Molecular weight: 1248,49 g/mol. HRMS m/z 1270,5310/1270,3177 (M+Na)⁺.

### Step 10. Synthesis of Suc-Phe-Val-Thr-(4-Gu)Phg^{P}(O-C₆H₄-4-S-CH₃)₂.

In the round-bottom flask product obtained in Step 9 (320 mg) is dissolved in 50% trifluoroacetic acid in dichloromethane (5ml). The reaction is performed at room temperature for 2h. After the reaction is completed volatile elements are removed in vacuum and the resulting oil is dissolved in toluene (5ml) and evaporated to dryness. Next, the product is purified by HPLC using water/acetonitrile system with 0,05% of TFA on C-18 column. Fractions containing desired product are freeze-dried leading to 42mg (18%). Empirical formula: C₄₄H₅₄N₇O₁₀PS₂. Molecular weight: 936,04 g/mol. Retention time (HPLC, SunFire™ Prep, 10x250 mm, 5 µm column, gradient 5%-95% MeCN/45min): 23,41 min. HRMS m/z 936,3190/936,0670 (M+H)⁺.

### Step 11. Sznthesis of Bt-LC-Suc-Phe-Val-Thr-(4-Gu)Phg^{P}(O-C₆H₄-4-S-CH₃)₂.

In round+bottom flask product obtained in Step 10 (10 mg) is dissolved in 0,5 ml of N-methylopyrrolidone and biotin pentyloamine (Bt-LC-NH₂) is added (3,5 mg, 0,01mmol) followed by the addition of DIEA (6µl, 0,03mmol). After complete dissolving of reagents 4,6mg (0,012mmol) of HBTU is added. The reaction is performed at room temperature for 18h. Next, the product is purified directly by HPLC using water/acetonitrile system with 0,05% of TFA on C-18 column. Fractions containing desired product are freeze-dried leading to 6,8 mg (54%). Empirical formula: C₅₉H₈₀N₁₁O₁₁PS₃. Molecular weight: 1246,50 g/mol. Retention time (HPLC, Alltech Econosphere C-18 10U, 250x22mm column, gradient 5%-95% MeCN/45min): 23,97 min. HRMS *m*/*z* 1246,5017/1246,4969 (M+H)⁺.

### Example 12

The method for preparation of bis(4-(methylthio)phenyl) (3-methyl-1-(1-(3-methyl-2-(5-(2-oxohexahydro-1H-thieno[3,4-d]imidazol-4-yl)pentanamido)butanoyl)pyrrolidine-2-carboxamido)butyl)phosphonate [Bt-Val-Pro-Leu^{P}(O-C₆H₄-4-SCH₃)₂] represented by the formula 12.

### Steps from 1 to 5 were described in example 8.

### Step 6. Synthesis of tripeptidyl derivative of di-4-thiomethylphenyl ester of

### 1-amino-3-methylbutane phosphonic acid.

Into the product obtained in Step 5 - TFA*Pro-Leu^{P}(OC₆H₄-4-S-CH₃)₂ (0,99g; 1,59mmol) 0,35g (1,59mmol) of Boc-Val-OH is added. After 2 minutes from complete dissolving in acetonitrile (15ml), 0,53ml of triethylamine (3,98 mmol) is added. After 10 minutes 0,64g (1.7mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 1.02g (91%). Empirical Formula: C₃₄H₅₀N₃O₇PS₂. Molecular weight: 707,88g/mol. ³¹P NMR (CDCl₃, ppm): δ 18,32 (s, 60%), 19,40 (s, 60%). ¹H NMR (CDCl₃, ppm): 80,94-1,02 (m, 12H), 1,36 (s, 1H), 1,50 (s, 9H), 1,60-1,92 (m, 7H), 2,35-2,38 (m, 6H), 3,50-3,55 (m, 2H), 4,50-4,52 (m, 1H), 4,80-4,90 (m, 1H), 5,10 (s, 1H), 6,20-7,50 (m, 8H). HRMS m/s 730,2725/730,2731 (M+Na)⁺.

### Step 7. Deprotection of t-Boc group in tripeptidyl derivative of di-4-thiomethylphenyl ester of 1-amino-3-methylbutane phosphonic acid.

Tripeptidyl derivative of di-4-thiomethylphenyl ester of 1-amino-3-methylbutane phosphonic acid is placed in the flask (1.0 g, 1,41 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,96 g which is used without purification in the next step for the synthesis of biotinylated tripeptidyl derivative of di-4-thiomethylphenyl ester of 1-amino-3-methylbutane phosphonic acid.

### Step 8. Synthesis of biotinylated tripeptidyl derivative of di-4-thiomethylphenyl ester of 1-amino-3-methylbutane phosphonic acid.

Product obtained in step TFA*Val-Pro-Leu^{P}(OC₆H₄-4-*S*-CH₃)₂ (0,96g, 1,33mmol) is dissolved in N-methylpyrrolidone (10ml) followed by the addition of biotin (0,32g, 1,33mmol). Next, N,N'-diisopropylamine (DIEA) is added in the amount of 0,58ml (3,33mmol). After complete dissolving 0,55g (1,46mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,49g, (44%). Empirical Formula: C₃₉H₅₆N₅O₇PS₃. Molecular weight: 833,31g/mol. Retention time (HPLC, Alltech Econosphere C-18 10U column, 250x22mm, gradient 5%-95% MeCN/45min): 22.46 min. HRMS m/z 834,3158/834,2836 (M+H)⁺.

### Example 13

The method for preparation of bis(4-(methylthio)phenyl) (1-(1-(3-methyl-2-(5-(2-oxohexahydro-1H-thieno[3,4-d]imidazol-4-yl)pentanamido)butanoyl)pyrrolidine-2-carboxamido)propyl)phosphonate [Bt-Val-Pro-Abu^{P}(O-C₆H₄-4-*S*-CH₃)₂] represented by the formula 13.

### Step 1. Synthesis of tri-(4-thiomethyl)phenyl phosphite.

In the round-bottom flask equipped with a magnetic stirrer 4,2g (30mmol) of 4-thiomethylphenol is dissolved in acetonitrile (20ml) followed by the addition of phosphorus (III) chloride (0,87ml, 10mmol). The reaction mixture is then refluxed for 6h. The volatile elements of the mixture are removed under reduced pressure and resulting product obtained as pale yellow oil is used without purification for the synthesis of di-4-thiomethylphenyl ester of N-(benzyloxycarbonyl-1-amino)-propanephosphonic acid.

### Step 2. Synthesis of di-4-thiomethylphenyl ester of N-(benzyloxycarbonyl-1-amino)-propanephosphonic acid.

Into the flask containing tri-(4-thiomethyl)phenyl phosphite obtained in step 1, 1,51g (10mmol) of benzyl carbamate and 0,86ml (12mmol) of propionaldehyde are added followed by the addition of glacial acetic acid (10 ml). The reaction mixture is heated at 90°C for 3h and the volatile components of the mixture are removed under reduced pressure. The product obtained as yellow oil is dissolved in 100-150ml of methanol and stored at -20°C for crystallization (24h). Crystallized product is collected by suction and air-dried obtaining 3,99g (77% yield). Empirical Formula: C₂₅H₂₈NO₅PS₂. Molecular weight: 517,11g/mol. ³¹P NMR (121MHz, CDCl₃, ppm): δ 18,79 (s); ¹H NMR (300 MHz, CDCl₃, ppm): δ 1,06-1,11 (t, *J* = 14,7 Hz, 3H), 1,72-1,80 (dd, *J* = 15,2/7,5 Hz, 1H,), 2,06-2,11 (m, 2H), 2,44 (s, 6H), 4,35-4,43 (m, 1H), 5,08-5,18 (d, *J* = 6,6 Hz, 2H). 7,00-7,33 (m, 13H). mp: 107°C. R_{f} = 0,60 (CHCl₃:AcOEt, 4:1, v/v).

### Step 3. Synthesis of di-4-thiomethylphenyl ester of 1-amino-propanephosphonic acid hydrobromide salt.

Into the round-bottom flask di-4-thiomethylphenyl ester of N-(benzyloxycarbonyl-1-amino)-propanephosphonic acid (2,00g, 3,87 mmol) is added and dissolved in 5,00 ml of 33% solution of hydrobromic acid in acetic acid. The reaction is performed at room temperature for 2h. The volatile components are removed in vacuum and resulting oil is dissolved in minimal volume of methanol followed by the addition of diethyl ether until first sign of precipitation. The solution is placed at -20°C for 24 h to complete crystallization of the product which is then collected by suction, air-dried leading to 1,67 g (93% yield) of di-4-thiomethylphenyl ester of 1-amino-propanephosphonic acid hydrobromide salt. Empirical Formula: C₁₇H₂₃BrNO₃PS₂. Molecular weight: 463,00g/mol. ³¹P NMR (121MHz, DMSO-d₆, ppm): δ 13,03 (s); ¹H NMR (300 MHz, DMSO-d₆, ppm): δ 1,17-1,22 (t, *J* = 6,3 Hz, 3H), 2,08-2,20 (m, 2H), 2,37 (s, 6H), 3,95-4,02 (m, 1H), 7,00-7,23 (m, 8H), 8,92-9,09 (d, *J* = 6,9 Hz, 3H). mp: 148°C. R_{f} = 0,21 (CHCl₃:AcOEt, 4:1, v/v).

### Step 4. Synthesis of dipeptidyl derivative of di-4-thiomethylphenyl ester of 1-amino-propanephosphonic acid.

Into the round-bottom flask 0,93g (2 mmol) of di-4-thiomethylphenyl ester of 1-amino-propanephosphonic acid hydrobromide salt is added followed by the addition of 0,43g Boc-Pro-OH (2mmol). After 2 minutes from complete dissolving in acetonitrile (15ml), 0,55ml of triethylamine (4mmol) is added. After 10 minutes 0,76g (2mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4: 1) as the eluent. The product is obtained as colorless oil, 1,01g (87%). Empirical Formula: C₂₇H₃₇O₆N₂PS₂. Molecular weight: 580,18g/mol. ³¹P NMR (121MHz, CDCl₃, ppm): δ 18,95 (s). ¹H NMR (300 MHz, CDCl₃, ppm): δ 0,96-1,08 (m, 2H), 1,22-1,27 (m, 2H), 1,43 (s, 9H), 1,60-2,40 (m, 5H), 2,44 (s, 6H). mp: 54°C. R_{f} = 0,28 (CHCl₃:AcOEt; 4:1, v/v).

### Step 5. Deprotection of t-Boc group in dipeptidyl derivative of di-4-thiomethylphenyl ester of 1-amino-propanephosphonic acid.

Dipeptidyl derivative of di-4-thiomethylphenyl ester of 1-amino-propanephosphonic acid is placed in the flask (1,0 g, 1,72 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,96g which is used without purification in the next step for the synthesis of tripeptidyl derivative of di-4-thiomethylphenyl ester of 1-amino-propanephosphonic acid.

### Step 6. Synthesis of tripeptidyl derivative of di-4-thiomethylphenyl ester of 1-amino-propanephosphonic acid.

Into the product obtained in Step 5 - TFA*Pro-Abu^{P}(OC₆H₄-4-S-CH₃)₂ (m=0,96g; 1,62mmol) 0,36g (1,62mmol) of Boc-Val-OH is added. After 2 minutes from complete dissolving in acetonitrile (15ml), 0,54ml of triethylamine (4mmol) is added. After 10 minutes 0,68g (1.8mmol) of HBTU is added and the reaction is performed at room temperature for 24h. The reaction mixture is extracted 3-times with ethyl acetate and organic fraction is washed with KHSO₄aq (5%), brine, NaHCO₃aq (5%), and brine and dried over magnesium sulfate for 2h. Next, the solution is concentrated in vacuum and the product is purified on silica gel column using chloroform/ethyl acetate (4:1) as the eluent. The product is obtained as colorless oil, 0,97g (88%). Empirical Formula: C₃₂H₄₆N₃O₇PS₂. Molecular weight: 679,25g/mol. ³¹P NMR (CDCl₃, ppm): 18,66 (s, 59%), 19,06 (s, 41%). ¹H NMR (CDCl₃, ppm): δ 0,85-1,05 (m, 10H), 1,43 (s, 9H), 1,60-2,18 (m, 7H), 2,45 (s, 6H), 3,52-3,80 (m, 2H), 4,25-4,30 (m, 1H), 4,50-4,52 (d, *J*=6,0 Hz, 1H), 4,55-4,75 (m, 1H), 5,16-5,30 (dd, *J* = 9,0/33,0 Hz, 1H), 7,03-7,22 (m, 8H). mp: 53°C. R_{f} = 0,10 (CHCl₃: AcOEt; 4:1, v/v).

### Step 7. Deprotection of t-Boc group in tripeptidyl derivative of di-4-thiomethylphenyl ester of 1-amino-propanephosphonic acid.

Tripeptidyl derivative of di-4-thiomethylphenyl ester of 1-amino-propanephosphonic acid is placed in the flask (0,95 g, 1,40 mmol) and 10ml of 50% trifluoroacetic acid in dichloromethane is added. The reaction is performed at room temperature and its progress is monitored by TLC. After the reaction is completed volatile elements are removed in vacuum and the resulting oil is dissolved in toluene (5ml) and evaporated to dryness. The product is obtained as pale yellow oil 0,93g which is used without purification in the next step for the synthesis of biotinylated tripeptidyl derivative of di-4-thiomethylphenyl ester of *1-amino-propanephosphonic* acid.

### Step 8. Synthesis of biotinylated tripeptidyl derivative of di-4-thiomethylphenyl ester of 1-amino-propanephosphonic acid.

Product obtained in step 7 TFA*Val-Pro-Abu^{P}(OC₆H₄-4-*S*-CH₃)₂ (0,93g, 1,34mmol) is dissolved in N-methylpyrrolidone (10ml) followed by the addition of biotin (0,33g, 1,34mmol). Next, N,N'-diisopropylamine (DIEA) is added in the amount of 0,58ml (3,35mmol). After complete dissolving 0,56g (1,47mmol) of HBTU is added. The reaction is performed at room temperature for 18 h. The product is then purified directly on HPLC using water/acetonitrile system with 0,05% of TFA on C-18 column. Fractions containing desired product are freeze-dried leading to 0,61g, (57%). Empirical Formula: C₃₇H₅₂N₅O₇PS₃. Molecular weight: 805,28g/mol. Retention time (HPLC, Alltech Econosphere C-18 10U column, 250x22mm, gradient 5%-95% MeCN/45min): 20.55 min. HRMS *m*/*z* 828,2664/828,2648 (M+Na)⁺.

### Example 14

The inhibitory activity of the derivatives obtained in Examples 1-13 against selected serine and cysteine proteases.

The biological activity of peptidyl derivatives of phosphonic analogs of valine, alanine, phenylalanine, 4-guanidinephenylglycine, leucine, and 1-aminobutyric acid, containing the N-terminal biotin residue were tested for their ability to inhibit proteolytic activity of selected serine proteases, and the control cysteine proteases. All tests were performed using a microplate reader. As enzyme substrates fluorescent peptides were used. The method to measure the activity of the compounds obtained are described below for each of the enzymes tested: human neutrophil elastase (HNE), porcine pancreatic elastase (PPE), subtilisin (Sub), cathepsin G (CatG), proteinase 3 (PR3), chymotrypsin (ChTry) trypsin (Try), papain (Pap) and cathepsin L (CatL). Measurement of the inhibitory activity was performed at different inhibitor concentrations ranging from 12.5 nM to 2000nm.

### Measurement of inhibitory activity of compounds toward human neutrophil elastase (HNE).

The inhibitory activity assay is performed at 37°C in 0,01M HEPES, 0,5M NaCl, 0,03% Triton X-100 buffer, pH 7,5, within 10 min after initial pre-incubation of the enzyme with inhibitor tested at 37°C for 10 min. The enzyme concentration is 0,0025U/ml. Substrate used Me-Suc-Ala-Ala-Pro-Val-AFC at 25µM. Measurement is taken at an excitation wavelength of 400nm and 505nm emission.

### Measurement of inhibitory activity of compounds toward porcine pancreatic elastase (PPE).

The inhibitory activity assay is performed at 37°C in 0,01M Tris-HCl, 0,05% Triton X-100 buffer, pH 8,5, within 10 min after initial pre-incubation of the enzyme with inhibitor tested at 37°C for 10 min. The enzyme concentration is 60nM. Substrate used Me-Suc-Ala-Ala-Pro-Val-AMC at 100µM. Measurement is taken at an excitation wavelength of 350nm and 460nm emission.

### Measurement of inhibitory activity of compounds toward subtilisin (Sub).

The inhibitory activity assay is performed at 37°C in 0,05M Tris-HCl, 1M NaCl, 0,01% Triton X-100 buffer, pH 7,5, within 10 min after initial pre-incubation of the enzyme with inhibitor tested at 37°C for 10 min. The enzyme concentration is 5nM. Substrate used Me-Suc-Ala-Ala-Pro-Phe-AMC at 5µM. Measurement is taken at an excitation wavelength of 350nm and 460nm emission.

### Measurement of inhibitory activity of compounds toward trypsin (Try).

The inhibitory activity assay is performed at 37°C in 0,1M HEPES, 0,5M NaCl, 0,03% Triton X-100 buffer, pH 7,5, within 10 min after initial pre-incubation of the enzyme with inhibitor tested at 37°C for 10 min. The enzyme concentration is 25nM. Substrate used Cbz-Arg-AFC at 100µM. Measurement is taken at an excitation wavelength of 400nm and 505nm emission.

### Measurement of inhibitory activity of compounds toward trypsin (ChyTry).

The inhibitory activity assay is performed at 37°C in 0,1M HEPES, 0,5M NaCl buffer, pH 7,5, within 10 min after initial pre-incubation of the enzyme with inhibitor tested at 37°C for 10 min. The enzyme concentration is 3nM. Substrate used Suc-Ala-Ala-Pro-Phe-AMC at 5µM. Measurement is taken at an excitation wavelength of 350nm and 460nm emission.

### Measurement of inhibitory activity of compounds toward cathepsin G (CatG).

The inhibitory activity assay is performed at 37°C in 0,1M Tric-HCl, 0,5M NaCl buffer, pH 7,5, within 10 min after initial pre-incubation of the enzyme with inhibitor tested at 37°C for 10 min. The enzyme concentration is 100nM. Substrate used MCA-Phe-Val-Thr-Gnf-Ser-Trp-Anb-NH₂ at 2.5µM. Measurement is taken at an excitation wavelength of 325nm and 400nm emission.

### Measurement of inhibitory activity of compounds toward proteinase 3(PR3).

The inhibitory activity assay is performed at 37°C in 0,1M Tric-HCl, 0,5M NaCl buffer, pH 7,5, within 10 min after initial pre-incubation of the enzyme with inhibitor tested at 37°C for 10 min. The enzyme concentration is 17nM. Substrate used Abz-Tyr-Tyr-Abu-Anb-NH₂ at 30µM. Measurement is taken at an excitation wavelength of 325nm and 400nm emission.

### Measurement of inhibitory activity of compounds toward cathepsin L(CatL).

The inhibitory activity assay is performed at 37°C in 0,4M sodium acetate, 4mM EDTA, 8mM DTT buffer, pH 5,5, within 10 min after initial pre-incubation of the enzyme with inhibitor tested at 37°C for 10 min. The enzyme concentration is 2ng/ml. Substrate used Cbz-Phe-Arg-AMC at 7µM. Measurement is taken at an excitation wavelength of 350nm and 460nm emission.

### Measurement of inhibitory activity of compounds toward papain (Pap).

The inhibitory activity assay is performed at 37°C in 0,4M sodium acetate, 4mM EDTA, 8mM DTT buffer, pH 5,5, within 10 min after initial pre-incubation of the enzyme with inhibitor tested at 37°C for 10 min. The enzyme concentration is 2ng/ml. Substrate used Cbz-Phe-Arg-AMC at 7µM. Measurement is taken at an excitation wavelength of 350nm and 460nm emission.

The results are shown in Table 1. The IC₅₀ value is the concentration of inhibitor required to inhibit half of the enzyme activity after 10 minutes incubation.

| | **HNE** | | **PPE** | | **PR3** | | **Cat G** | |
|---|---|---|---|---|---|---|---|---|
| | IC₅₀ [nM] | K_{obs}/[I] [M⁻¹S⁻¹] | IC₅₀ [nM] | K_{obs}/[I] [M⁻¹S⁻¹] | IC₅₀ [nM] | K_{obs}/[I] [M⁻¹S⁻¹] | IC₅₀ [nM] | K_{obs}/[I] [M⁻¹S⁻¹] |
| **1** | 2.1±0.1 | 5.5*10⁵ | 23.5±4.5 | 4.9*10⁴ | 72.5±6 | 3.2*10⁴ | NI | NI |
| **2** | 6.5±0.3 | 1.8*10⁵ | 245.5±30.5 | 4.7*10³ | 728±44 | 3.2*10³ | NI | NI |
| **3** | NI | NI | NI | NI | NI | NI | 258±33 | 4.5*10³ |
| **4** | 5.55±0.25 | 2.1*10⁵ | 135.5±7.5 | 8.5*10³ | NI | NI | NI | NI |
| **5** | NI | NI | 5000 | 2*10² | NI | NI | NI | NI |
| **6** | 1.55±0.05 | 2.5*10⁵ | NI | NI | NI | NI | 218±45 | 5.3*10³ |
| **7** | NI | NI | NI | NI | NI | NI | NI | NI |
| **8** | 3797±250 | 3.0*10² | NI | NI | NI | NI | 1095±139 | 1.0*10³ |
| **9** | 5.61±0.02 | 2.1*105 | >5000 | <200 | 59.5±8 | 3.9*10⁴ | 720±60 | 1.6*10³ |
| **10** | NI | NI | NI | NI | NI | NI | 300±0.5 | 3.8*10³ |
| **11** | NI | NI | NI | NI | 603±49 | 3.8*10³ | 4741±556 | 2.4*10² |
| **12** | 66.5±6.5 | 1.7*10⁴ | 288.8±10 | 4*10³ | NI | NI | 436±22 | 2.6*10³ |
| **13** | 2.75±0.05 | 4.2*10⁵ | 23.5±1.95 | 4.9*104 | 227±17 | 1.0*10⁴ | NI | NI |

| | **ChyTr** | | **Sub** | | **Try** | | **Cat L** | | **Pap** | |
|---|---|---|---|---|---|---|---|---|---|---|
| | IC₅₀ [nM] | K_{obs}/[I] [M⁻¹S⁻¹] | IC₅₀ [nM] | K_{obs}/[I] [M⁻¹S⁻¹] | IC₅₀ [nM] | K_{obs}/[I] [M⁻¹S⁻¹] | IC₅₀ [nM] | K_{obs}/[I] [M⁻¹S⁻¹] | IC₅₀ [nM] | K_{obs}/[I] [M⁻¹S⁻¹] |
| **1** | 182.5±3.5 | 6.3*10³ | 77.5±4.5 | 1.5*10⁴ | NI | NI | NI | NI | NI | NI |
| **2** | NI | NI | 415±17 | 2.8*10³ | NI | NI | NI | NI | NI | NI |
| **3** | 20.65±0.25 | 5.6*10⁴ | 11.6±0.2 | 1.0*10⁵ | NI | NI | NI | NI | NI | NI |
| **4** | NI | NI | 40.2±0.01 | 2.9*10⁴ | NI | NI | NI | NI | NI | NI |
| **5** | 156±12 | 7.4*10³ | 47.9±0.57 | 2.4*10⁴ | NI | NI | NI | NI | NI | NI |
| **6** | 11.5±2.5 | 1.0*10⁵ | 5.6±0.8 | 2.1*105 | 3150±550 | 3.7*10² | NI | NI | NI | NI |
| **7** | 123.3±4.4 | 9.4*10³ | 76.5±1.65 | 1.5*10⁴ | NI | NI | NI | NI | NI | NI |
| **8** | 400±30 | 2.9*10³ | 11.7±0.61 | 9.9*10⁴ | 1150±140 | 1*10³ | NI | NI | NI | NI |
| **9** | 22±4 | 5.2*10⁴ | 21.2±1.2 | 5.4*10⁴ | 26.25±0.45 | 4.4*10⁴ | NI | NI | NI | NI |
| **10** | 16.4±1.2 | 7.0*10⁴ | 39.95±4.6 | 2.9*10⁴ | NI | NI | NI | NI | NI | NI |
| **11** | NI | NI | 480.5±15.5 | 2.4*10³ | 424.5±9.5 | 2.7*10³ | NI | NI | NI | NI |
| **12** | 25.7±5 | 4.5*10⁴ | NI | NI | 612±90 | 1.9*10³ | NI | NI | NI | NI |
| **13** | 970±80 | 1.2*10³ | 147±4 | 7.8*10³ | 3900±200 | 3*10² | NI | NI | NI | NI |

| | | | | | | | | | | |
|---|---|---|---|---|---|---|---|---|---|---|
| NI- brak inhibicji | | | | | | | | | | |

Table 1. The results of biological activity screening of peptidyl derivatives of phosphonic analogs of valine, alanine, phenylalanine, 4-guanidinephenylglycine, leucine, and 1-aminobutyric acid, containing the N-terminal biotin residue toward human neutrophil elastase (HNE), porcine pancreatic elastase (PPE), subtilisin (Sub), cathepsin G (CatG), proteinase 3 (PR3), chymotrypsin (ChTry) trypsin (Try), papain (Pap) and cathepsin L (CatL).

### Example 15.

### Detection of enzymatically active subtilisin in Bacillus subtilis culture medium.

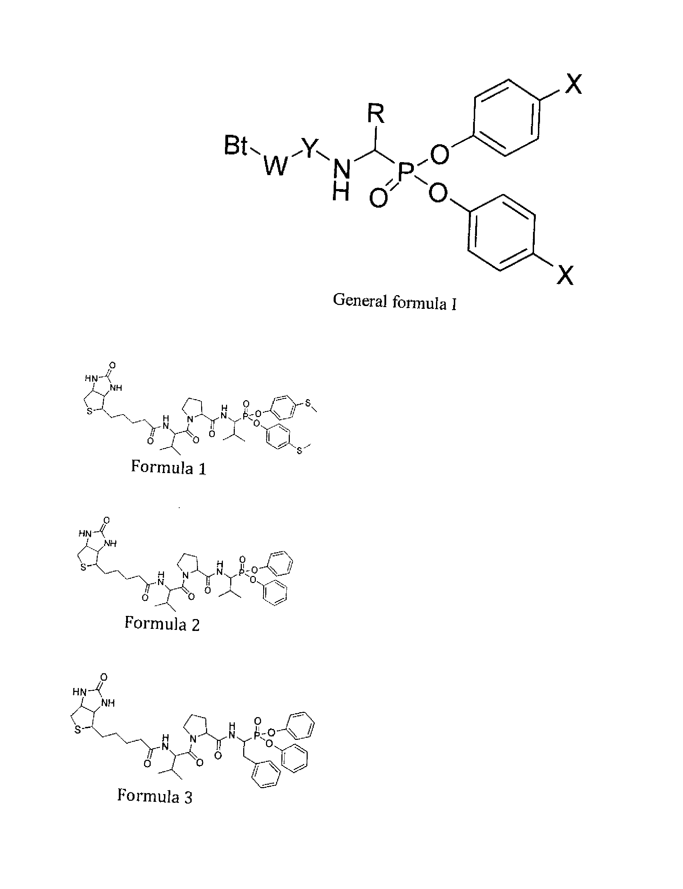

From 48h culture of *Bacillus subtilis* 10 ml of medium was taken and filtered through the syringe filter (0,22µm) in order to remove cells. Into the filtered solution (50µl) 1 µl of tested inhibitor solution (into each tube different inhibitor was added) at 1mM in DMSO. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 1.

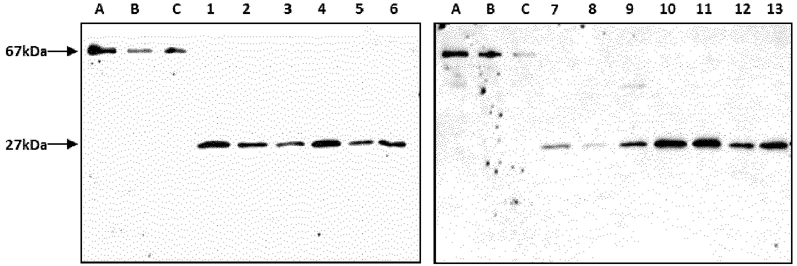
A B C 1 2 3 4 5 6
67kDa
27kDa
A B C 7 8 9 10 11 12 13

Fig. 1. Western blotting analysis of *Bacillus subtilis* culture medium after incubation with obtained compounds. Abbr.: A, B, C - positive control - biotinylated bovine albumin of 2.4 (**A**), 1.2 (**B**) and 0.6 (**C**) pmol of biotin/lane. Numbers 1-13 correspond to synthesized compounds: **1** [Bt-Val-Pro-Val^{P}(OC₆H₄-4-S-CH₃)₂; Example 1], **2** [Bt-Val-Pro-Val^{P}(OC₆H₅)₂; Example 2], **3** [Bt-Val-Pro-Phe^{P}(OC₆H₅)₂; Example 3], **4** [Bt-Val-Pro-Ala^{P}(OC₆H₄-4-S-CH₃)₂; Example 4], **5** [Bt-Val-Pro-Leu^{P}(OC₆H₄-4-O-CH₃)₂; Example 5], **6** [Bt-Val-Pro-Phe^{P}(OC₆H₄-4-S-CH₃)₂; Example 6], **7** [Bt-Val-Pro-Leu^{P}(OC₆H₅)₂; Example 7], **8** [Bt-Pro-Leu^{P}(OC₆H₄-4-S-CH₃)₂; Example 8], **9** [Bt-Val-Pro-Leu^{P}(OC₆H₄-4-COOCH₃)₂; Example 9], **10** [Bt-LC-Suc-Val-Pro-Phe^{P}(OC₆H₅)₂; Example 10], **11** [Bt-LC-Suc-Phe-Val-Thr-(4-GuPhg)^{P}(OC₆H₄-4-S-CH₃)₂; Example 11], **12** [Bt-Val-Pro-Leu^{P}(OC₆H₄-4-S-CH₃)₂; Example 12], **13** [Bt-Val-Pro-Abu^{P}(OC₆H₄-4-S-CH₃)₂; Example 13].

### Example 16.

### Detection of active subtilisin from Bacillus licheniformis.

The solution of subtilisin from *Bacillus licheniformis* (0,8187 mg/ml) was diluted with 10mM PBS to the final enzyme concentration of 62,5µg/ml. From resulted solution aliquots of 20µl were transferred into separate tubes followed by the addition of 1 µl of tested compound at 1mM in DMSO. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 2.

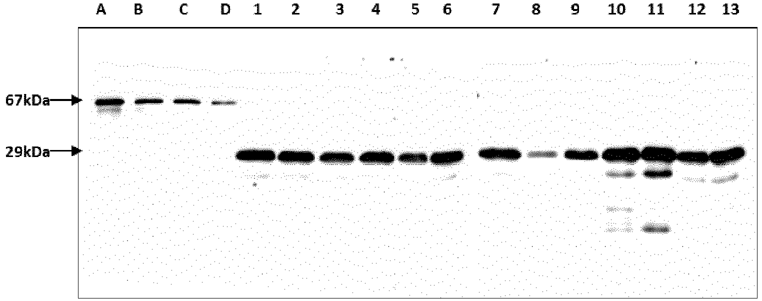
A B C D 1 2 3 4 5 6 7 8 9 10 11 12 13
67kDa
29kDa

Fig. 2. Western blotting analysis of subtilisin from *Bacillus licheniformis* after incubation with obtained compounds. Abbr.: A, B, C - positive control - biotinylated bovine albumin of 2.4 (**A**), 1.2 (**B**) and 0.6 (**C**) pmol of biotin/lane. Numbers 1-13 correspond to synthesized compounds: **1** [Bt-Val-Pro-Val^{P}(OC₆H₄-4-S-CH₃)₂; Example 1], **2** [Bt-Val-Pro-Val^{P}(OC₆H₅)₂; Example 2], **3** [Bt-Val-Pro-Phe^{P}(OC₆H₅)₂; Example 3], **4** [Bt-Val-Pro-Ala^{P}(OC₆H₄-4-S-CH₃)₂; Example 4], **5** [Bt-Val-Pro-Leu^{P}(OC₆H₄-4-O-CH₃)₂; Example 5], **6** [Bt-Val-Pro-Phe^{P}(OC₆H₄-4-S-CH₃)₂; Example 6], **7** [Bt-Val-Pro-Leu^{P}(OC₆H₅)₂; Example 7], **8** [Bt-Pro-Leu^{P}(OC₆H₄-4-S-CH₃)₂; Example 8], **9** [Bt-Val-Pro-Leu^{P}(OC₆H₄-4-COOCH₃)₂; Example 9], **10** [Bt-LC-Suc-Val-Pro-Phe^{P}(OC₆H₅)₂; Example 10], **11** [Bt-LC-Suc-Phe-Val-Thr-(4-GuPhg)^{P}(OC₆H₄-4-S-CH₃)₂; Example 11], **12** [Bt-Val-Pro-Leu^{P}(OC₆H₄-4-S-CH₃)₂; Example 12], **13** [Bt-Val-Pro-Abu^{P}(OC₆H₄-4-S-CH₃)₂; Example 13].

### Example 17

### Detection of enzymatically active cathepsin G.

The solution of cathepsin G (1.176 mg/ml) was diluted with 10mM PBS to the final enzyme concentration of 62,5µg/ml. From resulted solution aliquots of 20µl were transferred into separate tubes followed by the addition of 1 µl of tested compounds at 1mM in DMSO. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 3.

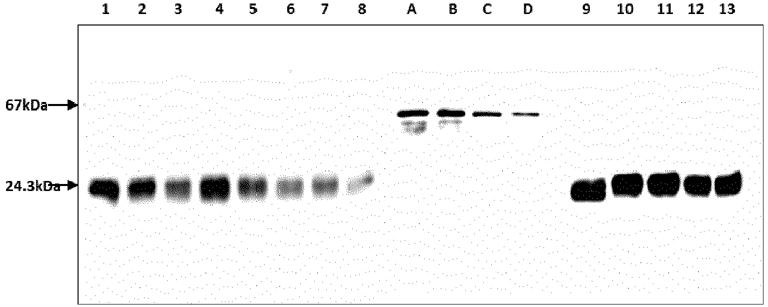
1 2 3 4 5 6 7 8 A B C D 9 10 11 12 13
67kDa
24.3kDa

Fig. 3. Western blotting analysis of cathepsin G after incubation with obtained compounds. Abbr.: A, B, C - positive control - biotinylated bovine albumin of 2.4 (**A**), 1.2 (**B**) and 0.6 (**C**) pmol of biotin/lane. Numbers 1-13 correspond to synthesized compounds: **1** [Bt-Val-Pro-Val^{P}(OC₆H₄-4-*S*-CH₃)₂; Example 1], **2** [Bt-Val-Pro-Val^{P}(OC₆H₅)₂; Example 2], **3** [Bt-Val-Pro-Phe^{P}(OC₆H₅)₂; Example 3], **4** [Bt-Val-Pro-Ala^{P}(OC₆H₄-4-*S*-CH₃)₂; Example 4], **5** [Bt-Val-Pro-Leu^{P}(OC₆H₄-4-O-CH₃)₂; Example 5], **6** [Bt-Val-Pro-Phe^{P}(OC₆H₄-4-S-CH₃)₂; Example 6], **7** [Bt-Val-Pro-Leu^{P}(OC₆H₅)₂; Example 7], **8** [Bt-Pro-Leu^{P}(OC₆H₄-4-S-CH₃)₂; Example 8], **9** [Bt-Val-Pro-Leu^{P}(OC₆H₄-4-COOCH₃)₂; Example 9], **10** [Bt-LC-Suc-Val-Pro-Phe^{P}(OC₆H₅)₂; Example 10], **11** [Bt-LC-Suc-Phe-Val-Thr-(4-GuPhg)^{P}(OC₆H₄-4-*S*-CH₃)₂; Example 11], **12** [Bt-Val-Pro-Leu^{P}(OC₆H₄-4-S-CH₃)₂; Example 12],**13** [Bt-Val-Pro-Abu^{P}(OC₆H₄-4-S-CH₃)₂; Example 13].

### Example 18

### Determination of the detection limit of the active cathepsin G using compound 4 [Bt-Val-Pro-Ala^{P}(OC₆H₄-4-S-CH₃)₂].

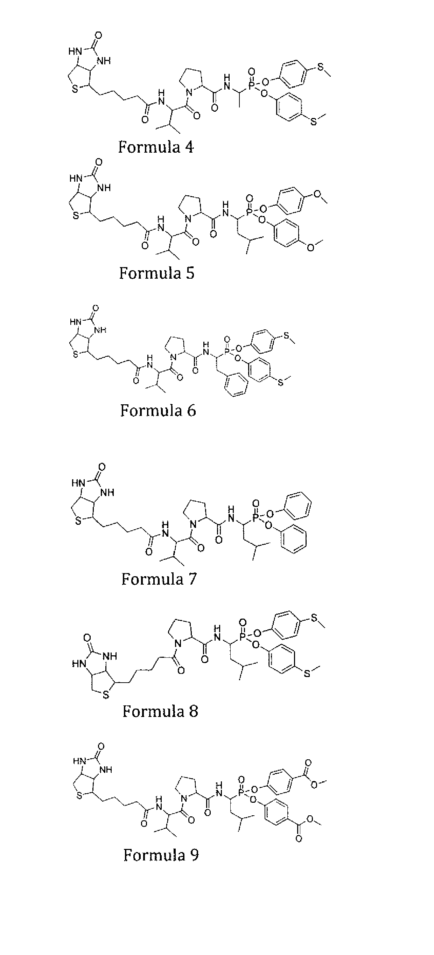

The solution of cathepsin G (1.176 mg/ml) was diluted with 10mM PBS to the final enzyme concentration of 100µg/ml, 50µg/ml, 25µg/ml, 12,5µg/ml, 6,25µg/ml, 3,125µg/ml, 1,5625µg/ml, 0,78125µg/ml, 0,390625µg/ml. From resulted solution aliquots of 20µl were transferred into separate tubes followed by the addition of 1 µl of tested compound. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 4.

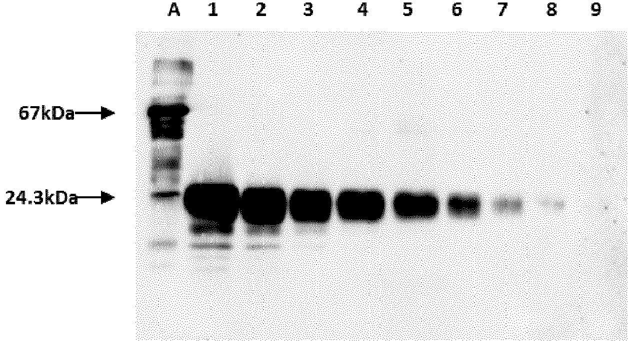
A 1 2 3 4 5 6 7 8 9
67kDa
24.3kDa

Fig. 4. Western blotting analysis of cathepsin G after incubation with compound Bt-Val-Pro-Ala^{P}(OC₆H₄-4-*S*-CH₃)₂.. Abbr.: A - positive control - biotinylated bovine albumin of 4.8 pmol of biotin/lane. Numbers 1-9 correspond to cathepsin G amount per lane incubated with tested conpound: **1 -** 1000ng; **2 -** 500ng; **3 -** 250ng; **4 -** 125ng; **5 -** 62,5ng; **6 -** 31,25ng; **7 -** 15,625ng; **8 -** 7,8125ng; **9 -** 3,90625ng.

### Example 19

### Detection of enzymatically active trypsin.

The solution of trypsin (1 mg/ml) was diluted with 10mM PBS to the final enzyme concentration of 10µg/ml. From resulted solution aliquots of 20µl were transferred into separate tubes followed by the addition of 1 µl of tested compounds at 1mM in DMSO. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 5.

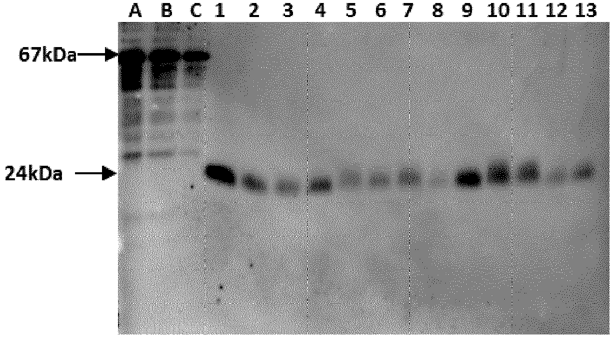
A B C 1 2 3 4 5 6 7 8 9 10 11 12 13
67kDa
24kDa

Fig. 5. Western blotting analysis of trypsin after incubation with obtained compounds. Abbr.: A, B, C - positive control - biotinylated bovine albumin of 2.4 (**A**), 1.2 (**B**) and 0.6 (**C**) pmol of biotin/lane. Numbers 1-13 correspond to synthesized compounds: **1** [Bt-Val-Pro-Val^{P}(OC₆H₄-4-S-CH₃)₂; Example 1], **2** [Bt-Val-Pro-Val^{P}(OC₆H₅)₂; Example 2], **3** [Bt-Val-Pro-Phe^{P}(OC₆H₅)₂; Example 3], **4** [Bt-Val-Pro-Ala^{P}(OC₆H₄-4-S-CH₃)₂; Example 4], **5** [Bt-Val-Pro-Leu^{P}(OC₆H₄-4-O-CH₃)₂; Example 5], **6** [Bt-Val-Pro-Phe^{P}(OC₆H₄-4-S-CH₃)₂; Example 6], **7** [Bt-Val-Pro-Leu^{P}(OC₆H₅)₂; Example 7], **8** [Bt-Pro-Leu^{P}(OC₆H₄-4-S-CH₃)₂; Example 8], **9** [Bt-Val-Pro-Leu^{P}(OC₆H₄-4-COOCH₃)₂; Example 9], **10** [Bt-LC-Suc-Val-Pro-Phe^{P}(OC₆H₅)₂; Example 10], **11** [Bt-LC-Suc-Phe-Val-Thr-(4-GuPhg)^{P}(OC₆H₄-4-S-CH₃)₂; Example 11], **12** [Bt-Val-Pro-Leu^{P}(OC₆H₄-4-S-CH₃)₂; Example 12],**13** [Bt-Val-Pro-Abu^{P}(OC₆H₄-4-S-CH₃)₂; Example 13].

### Example 20

### Detection of enzymatically active human neutrophil elastase.

The solution of human neutrophil elastase (0.625 mg/ml) was diluted with 10mM PBS to the final enzyme concentration of 40µg/ml. From resulted solution aliquots of 20µl were transferred into separate tubes followed by the addition of 1 µl of tested compounds at 1mM in DMSO. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 6.

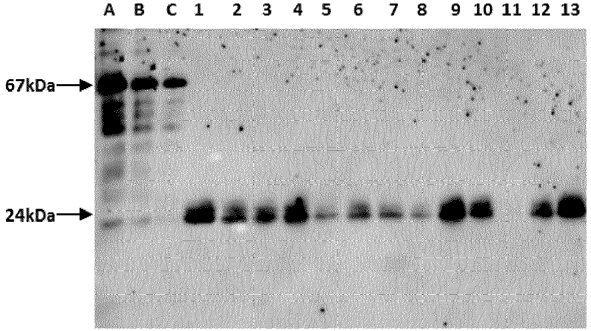
A B C 1 2 3 4 5 6 7 8 9 10 11 12 13
67kDa
24kDa

Fig. 6. Western blotting analysis of human neutrophil elastase after incubation with obtained compounds. Abbr.: A, B, C - positive control - biotinylated bovine albumin of 4.8 (**A**), 2.4 (**B**) and 1.2 (**C**) pmol of biotin/lane. Numbers 1-13 correspond to synthesized compounds: **1** [Bt-Val-Pro-Val^{P}(OC₆H₄-4-S-CH₃)₂; Example 1], **2** [Bt-Val-Pro-Val^{P}(OC₆H₅)₂; Example 2], **3** [Bt-Val-Pro-Phe^{P}(OC₆H₅)₂; Example 3], **4** [Bt-Val-Pro-Ala^{P}(OC₆H₄-4-S-CH₃)₂; Example 4], **5** [Bt-Val-Pro-Leu^{P}(OC₆H₄-4-O-CH₃)₂; Example 5], **6** [Bt-Val-Pro-Phe^{P}(OC₆H₄-4-S-CH₃)₂; Example 6], **7** [Bt-Val-Pro-Leu^{P}(OC₆H₅)₂; Example 7], **8** [Bt-Pro-Leu^{P}(OC₆H₄-4-S-CH₃)₂; Example 8], **9** [Bt-Val-Pro-Leu^{P}(OC₆H₄-4-COOCH₃)₂; Example 9], **10** [Bt-LC-Suc-Val-Pro-Phe^{P}(OC₆H₅)₂; Example 10], **11** [Bt-LC-Suc-Phe-Val-Thr-(4-GuPhg)^{P}(OC₆H₄-4-*S*-CH₃)₂; Example 11], **12** [Bt-Val-Pro-Leu^{P}(OC₆H₄-4-*S*-CH₃)₂; Example 12], **13** [Bt-Val-Pro-Abu^{P}(OC₆H₄-4-S-CH₃)₂; Example 13].

### Example 21

### Detection of enzymatically active porcine pancreatic elastase.

The solution of porcine pancreatic elastase (1 mg/ml) was diluted with 10mM PBS to the final enzyme concentration of 12.5µg/ml. From resulted solution aliquots of 20µl were transferred into separate tubes followed by the addition of 1 µl of tested compounds at 1mM in DMSO. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 7. 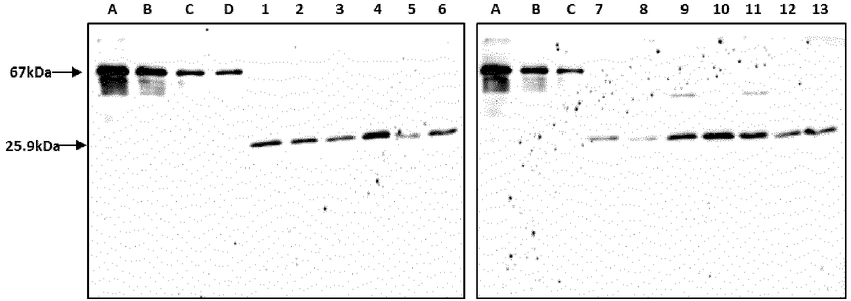
A B C D 1 2 3 4 5 6
A B C 7 8 9 10 11 12 13
67kDa
25.9kDa
 Fig. 7. Western blotting analysis of porcine pancreatic elastase after incubation with obtained compounds. Abbr.: A, B, C - positive control - biotinylated bovine albumin of 4.8 (**A**), 2.4 (**B**), 1.2 (**C**), and 0.6 (**D**) pmol of biotin/lane. Numbers 1-13 correspond to synthesized compounds: **1** [Bt-Val-Pro-Val^{P}(OC₆H₄-4-*S*-CH₃)₂; Example 1], **2** [Bt-Val-Pro-Val^{P}(OC₆H₅)₂; Example 2], **3** [Bt-Val-Pro-Phe^{P}(OC₆H₅)₂; Example 3], **4** [Bt-Val-Pro-Ala^{P}(OC₆H₄-4-*S*-CH₃)₂; Example 4], **5** [Bt-Val-Pro-Leu^{P}(OC₆H₄-4-O-CH₃)₂; Example 5], **6** [Bt-Val-Pro-Phe^{P}(OC₆H₄-4-S-CH₃)₂; Example 6], **7** [Bt-Val-Pro-Leu^{P}(OC₆H₅)₂; Example 7], **8** [Bt-Pro-Leu^{P}(OC₆H₄-4-S-CH₃)₂; Example 8], **9** [Bt-Val-Pro-Leu^{P}(OC₆H₄-4-COOCH₃)₂; Example 9], **10** [Bt-LC-Suc-Val-Pro-Phe^{P}(OC₆H₅)₂; Example 10], **11** [Bt-LC-Suc-Phe-Val-Thr-(4-GuPhg)^{P}(OC₆H₄-4-S-CH₃)₂; Example 11], **12** [Bt-Val-Pro-Leu^{P}(OC₆H₄-4-*S*-CH₃)₂; Example 12], **13** [Bt-Val-Pro-Abu^{P}(OC₆H₄-4-S-CH₃)₂; Example 13].

### Example 22

### Detection of enzymatically active human proteinase 3.

The solution of human proteinase 3 (1 mg/ml) was diluted with 10mM PBS to the final enzyme concentration of 12.5µg/ml. From resulted solution aliquots of 20µl were transferred into separate tubes followed by the addition of 1 µl of tested compounds at 1mM in DMSO. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 8.

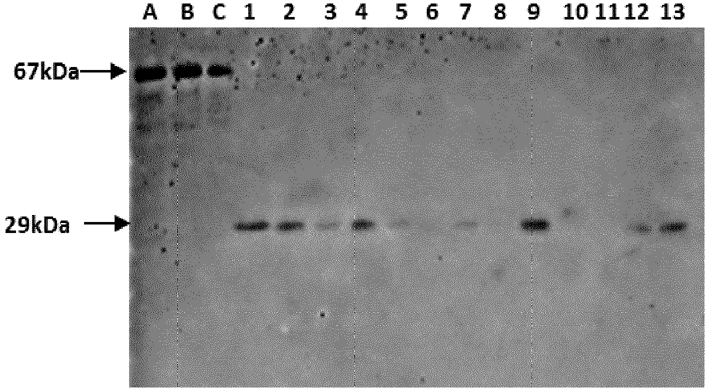
A B C 1 2 3 4 5 6 7 8 9 10 11 12 13
67kDa
29kDa

Fig. 8. Western blotting analysis of human proteinase 3 after incubation with obtained compounds. Abbr.: A, B, C - positive control - biotinylated bovine albumin of 4.8 (**A**), 2.4 (**B**) and 1.2 (**C**) pmol of biotin/lane. Numbers 1-13 correspond to synthesized compounds: **1** [Bt-Val-Pro-Val^{P}(OC₆H₄-4-S-CH₃)₂; Example 1], **2** [Bt-Val-Pro-Val^{P}(OC₆H₅)₂; Example 2], **3** [Bt-Val-Pro-Phe^{P}(OC₆H₅)₂; Example 3], **4** [Bt-Val-Pro-Ala^{P}(OC₆H₄-4-*S*-CH₃)₂; Example 4], **5** [Bt-Val-Pro-Leu^{P}(OC₆H₄-4-O-CH₃)₂; Example 5], **6** [Bt-Val-Pro-Phe^{P}(OC₆H₄-4-S-CH₃)₂; Example 6], **7** [Bt-Val-Pro-Leu^{P}(OC₆H₅)₂; Example 7], **8** [Bt-Pro-Leu^{P}(OC₆H₄-4-*S*-CH₃)₂; Example 8], **9** [Bt-Val-Pro-Leu^{P}(OC₆H₄-4-COOCH₃)₂; Example 9], **10** [Bt-LC-Suc-Val-Pro-Phe^{P}(OC₆H₅)₂; Example 10], 11 [Bt-LC-Suc-Phe-Val-Thr-(4-GuPhg)^{P}(OC₆H₄-4-*S*-CH₃)₂; Example 11], **12** [Bt-Val-Pro-Leu^{P}(OC₆H₄-4-*S*-CH₃)₂; Example 12], **13** [Bt-Val-Pro-Abu^{P}(OC₆H₄-4-*S*-CH₃)₂; Example 13].

Przyklad 23

### Detection of enzymatically active chymotrypsin.

The solution of chymotrypsin (1 mg/ml) was diluted with 10mM PBS to the final enzyme concentration of 62.5µg/ml. From resulted solution aliquots of 20µl were transferred into separate tubes followed by the addition of 1 µl of tested compounds at 1mM in DMSO. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 9. 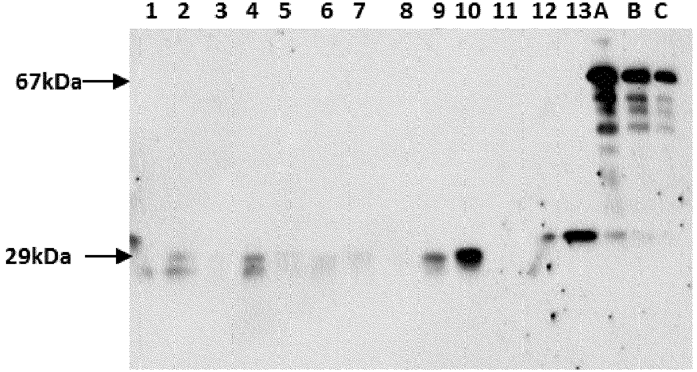
1 2 3 4 5 6 7 8 9 10 11 12 13A B C
67kDa
29kDa
 Fig. 9. Western blotting analysis of chymotrypsin after incubation with obtained compounds. Abbr.: A, B, C - positive control - biotinylated bovine albumin of 4.8 (**A**), 2.4 (**B**) and 1.2 (**C**) pmol of biotin/lane. Numbers 1-13 correspond to synthesized compounds: **1** [Bt-Val-Pro-Val^{P}(OC₆H₄-4-*S*-CH₃)₂; Example 1], **2** [Bt-Val-Pro-Val^{P}(OC₆H₅)₂; Example 2], **3** [Bt-Val-Pro-Phe^{P}(OC₆H₅)₂; Example 3], **4** [Bt-Val-Pro-Ala^{P}(OC₆H₄-4-S-CH₃)₂; Example 4], **5** [Bt-Val-Pro-Leu^{P}(OC₆H₄-4-O-CH₃)₂; Example 5], **6** [Bt-Val-Pro-Phe^{P}(OC₆H₄-4-*S*-CH₃)₂; Example 6], **7** [Bt-Val-Pro-Leu^{P}(OC₆H₅)₂; Example 7], **8** [Bt-Pro-Leu^{P}(OC₆H₄-4-S-CH₃)₂; Example 8], **9** [Bt-Val-Pro-Leu^{P}(OC₆H₄-4-COOCH₃)₂; Example 9], **10** [Bt-LC-Suc-Val-Pro-Phe^{P}(OC₆H₅)₂; Example 10], **11** [Bt-LC-Suc-Phe-Val-Thr-(4-GuPhg)^{P}(OC₆H₄-4-*S*-CH₃)₂; Example 11], **12** [Bt-Val-Pro-Leu^{P}(OC₆H₄-4-*S-*CH₃)₂; Example **12],13** [Bt-Val-Pro-Abu^{P}(OC₆H₄-4-*S*-CH₃)₂; Example 13].

### Example 24

Determination of the incubation time influence of Bt-LC-Suc-Val-Pro-Phe^{P}(OPh)₂ represented by the formula 10 with chymotrypsin on the complex detection by western blotting.

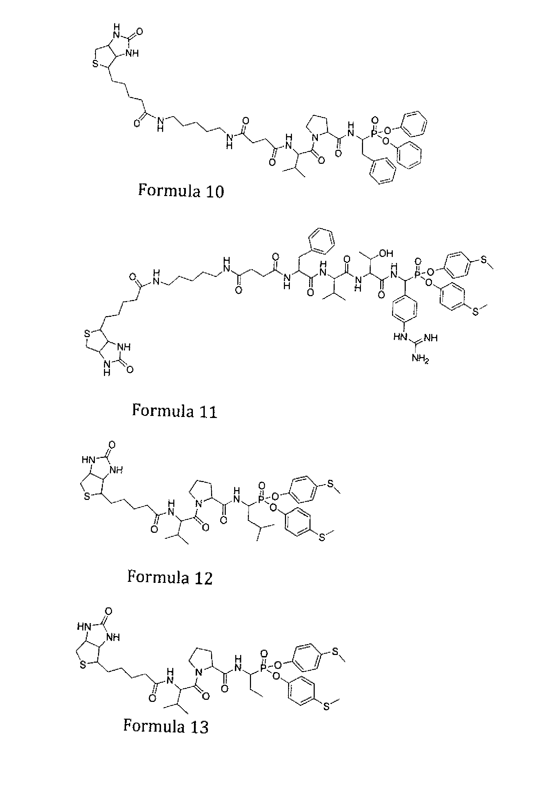

The solution of chymotrypsin (1 mg/ml) was diluted with 10mM PBS to the final enzyme concentration of 62.5µg/ml. From resulted solution 200µl was transferred into separate tube followed by the addition of 10 µl of tested compound at 1mM in DMSO. The mixtures were incubated at 37°C and 20µl aliquots were taken at different time-points (0, 5, 10, 15, 25, 30, 45 and 60 min). Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 10. 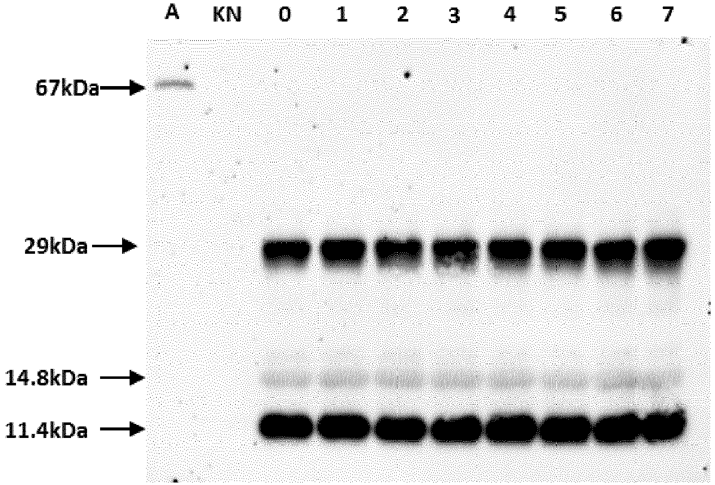
A KN 0 1 2 3 4 5 6 7
67kDa
29kDa
14.8kDa
11.4kDa
 Fig. 10. Western blotting analysis of chymotrypsin after incubation with tested compound at different time points. Abbr.: A - positive control - biotinylated bovine albumin of 2.4 pmol of biotin/lane, KN - negative control. Numbers 1-9 correspond to different time of incubation **0 -** 0min, **1 -** 5 min, **2 -** 10min, **3** - 15 min, **4 -** 25min, **5 -** 30min, **6 -** 45min, **7 -** 60min.

### Example 25

### Detection of enzymatically active papain.

The solution of papain (25.8 mg/ml) was diluted with acetate buffer (0.4M sodium acetate, 4mM EDTA, 8mM DTT) to the final enzyme concentration of 62.5µg/ml. From resulted solution aliquots of 20µl were transferred into separate tubes followed by the addition of 1 µl of tested compounds at 1mM in DMSO. The mixtures were incubated at 37°C for 1h. Into each tube electrophoresis reducing loading buffer was added and boiled for 10 minutes. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexes visualization was performed using molecular imaging system equipped with CCD camera using chemiluminescent peroxidase substrate. The obtained western blotting results are presented in Fig. 11. 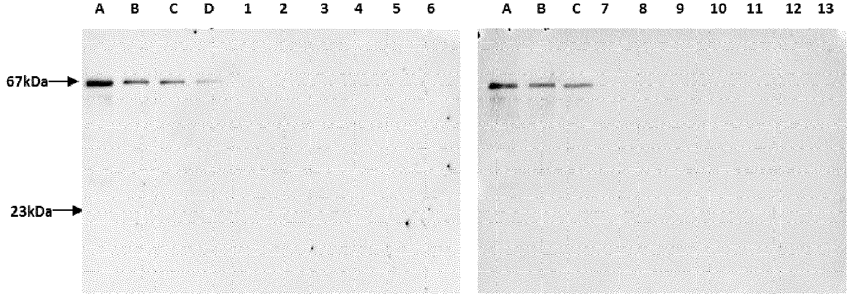
A B C D 1 2 3 4 5 6
67kDa
23kDa
A B C 7 8 9 10 11 12 13
 Fig. 7. Western blotting analysis of papain after incubation with obtained compounds. Abbr.: A, B, C - positive control - biotinylated bovine albumin of 4.8 (**A**), 2.4 (**B**), 1.2 (**C**), and 0.6 (**D**) pmol of biotin/lane. Numbers 1-13 correspond to synthesized compounds: **1** [Bt-Val-Pro-Val^{P}(OC₆H₄-4-*S*-CH₃)₂; Example 1], **2** [Bt-Val-Pro-Val^{P}(OC₆H₅)₂; Example 2], **3** [Bt-Val-Pro-Phe^{P}(OC₆H₅)₂; Example 3], **4** [Bt-Val-Pro-Ala^{P}(OC₆H₄-4-*S*-CH₃)₂; Example 4], **5** [Bt-Val-Pro-Leu^{P}(OC₆H₄-4-O-CH₃)₂; Example 5], **6** [Bt-Val-Pro-Phe^{P}(OC₆H₄-4-S-CH₃)₂; Example 6], **7** [Bt-Val-Pro-Leu^{P}(OC₆H₅)₂; Example 7], **8** [Bt-Pro-Leu^{P}(OC₆H₄-4-S-CH₃)₂; Example 8], **9** [Bt-Val-Pro-Leu^{P}(OC₆H₄-4-COOCH₃)₂; Example 9], **10** [Bt-LC-Suc-Val-Pro-Phe^{P}(OC₆H₅)₂; Example 10], **11** [Bt-LC-Suc-Phe-Val-Thr-(4-GuPhg)^{P}(OC₆H₄-4-*S*-CH₃)₂; Example 11], **12** [Bt-Val-Pro-Leu^{P}(OC₆H₄-4-*S*-CH₃)₂; Example 12], **13** [Bt-Val-Pro-Abu^{P}(OC₆H₄-4-*S*-CH₃)₂; Example 13].

### Example 26

### Detection of enzymatically active cathepsin G in human peripheral blood mononuclear cell lysates (PBMC).

Peripheral blood mononuclear cell were lysed using 10 mM Tris, 150 mM NaCl, 0.5% NP-40 buffer, pH 7,5. Next, from the solution 20µg of total protein was taken as determined by the Bradford assay and incubated in PBS buffer in the presence of compound **10** (final compound concentration in the mixture was 2µM). As the control commercially available probe Bt-DAP22c was used under identical conditions. The samples were then incubated at 37°C for 1h. Into each tube electrophoresis reducing loading buffer was added and boiled for 10 minutes. From each tube a volume corresponding to 20µg, 10µg or 5µg of total protein was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. As the loading control β-actin was detected by specific anti-β-actin mouse IgG and as the secondary antibody goat anti-mouse IgG-HRP conjugate was used. The complexes visualization was performed using autoradiography film using chemiluminescent peroxidase substrate. The obtained western blotting results are presented in Fig. 12. 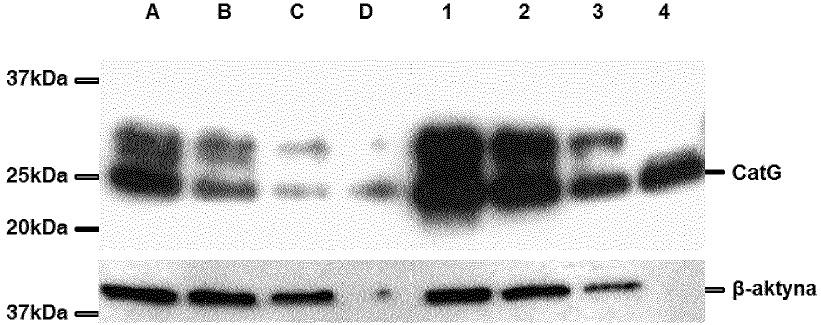
A B C D 1 2 3 4
37kDa
25kDa
20kDa
CatG
β-aktyna
37kDa
 Fig. 12. Western blotting analysis of human PBMC lysates after incubation with compound **10** [Bt-LC-Suc-Val-Pro-Phe^{P}(OC₆H₅)₂; Example 10]. Abrr.: A, B, C - positive control of lysates (**A** - 20µg, **B -** 10µg i **C -** 5µg) or pure cathepsin G (**D** - 25ng) incubated with commercial probe Bt-DAP22c; 1, 2, 3 - PBMC lysates (**1** - 20µg, **2 -** 10µg i **3 -** 5µg) or pure cathepsin G (**4** - 25ng) after incubation with compound **10.** Lower panel represents β-action loading control.

### Example 27

Determination of the subtilisin-Bt-LC-Suc-Phe-Val-Thr-(4-GuPhg)^{P}(OC₆H₄-4-*S*-CH₃)₂ complex stability.

The solution of subtilisin (0.8187 mg/ml) was diluted with 10mM PBS to the final enzyme concentration of 62.5µg/ml. From resulted solution 200µl was transferred into separate tube followed by the addition of 10 µl of tested compound at 1mM in DMSO. The mixtures were first incubated at 37°C for 1h and next incubated at room temperature. At different time points (0min - aliquot taken after 60 min incubation at 37°C; 6h, 12h, 24h, 48h, 36h, 60h, 72h) 20µl aliquots were taken. Into each tube electrophoresis reducing loading buffer was added and boiled for 10 minutes and stored at -20oC for further analysis. From each tube 10 µl was taken and separated on SDS PAGE (4-12%) followed by semi-dry blotting into nitrocellulose membrane. The membrane was blocked using 5% skim milk solution in PBS buffer containing 0.05% Tween-20. As the enzyme-inhibitor complex detection agent streptavidin-HRP conjugate was used. The complexed visualization was performed using molecular imaging system equipped with CCD camera using chemiluminescent peroxidase substrate. The obtained western blotting results are presented in Fig. 13. 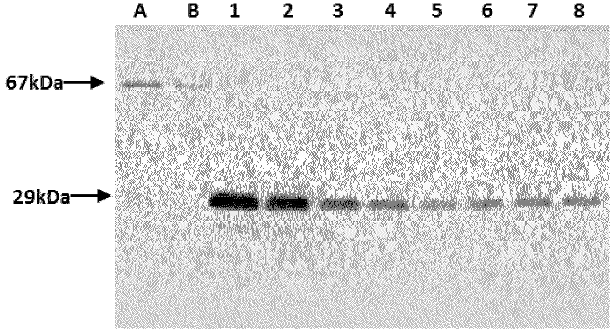
A B 1 2 3 4 5 6 7 8
67kDa
29kDa
 Fig. 13. Western blotting analysis of subtilisin-inhibitor complex stability at different time points. Abbr.: A, B - positive control - biotinylated bovine albumin of 2.4 (**A**) or 1.2 (**B**) pmol of biotin/lane. Numbers 1-8 correspond to different time of incubation: **1**- 0 min, **2** - 6h, **3** - 12h, **4** - 24h, **5** - 36h, **6** - 48h, **7** - 60h, 8 - 72h.

## Claims

1. Derivatives of 1-aminoalkylphosphonate diaryl esters of formula I, in which Bt represents biotin, W is a hydrocarbon linker chain of 5 to 10 carbon atoms, Y is the amino acid residues in an amount of 0 to 4, R is a substituent corresponding to the side chain of valine, alanine, leucine, phenylalanine, 4-guanidinephenylglycine, or 2-aminobutyric acid (Abu), X is the hydrogen or a compound selected from the group consisting of mercaptomethyl (S-methyl), methoxy (O-methyl), or carboxymethyl.

2. Derivative according to claim. 1 characterized as a mixture of diastereomers.

3. A process for preparation of 1-aminoalkylphosphonate diaryl esters of formula **I**, in which Bt represents biotin, W is a hydrocarbon linker chain of 5 to 10 carbon atoms, Y is the amino acid residues in an amount of 0 to 4, R is a substituent corresponding to the side chain of valine, alanine, leucine, phenylalanine, 4-guanidinephenylglycine, or 2-aminobutyric acid (Abu), X is the hydrogen or a compound selected from the group consisting of mercaptomethyl (S-methyl), methoxy (O-methyl), or carboxymethyl **characterized in that** triaryl phosphite, obtained from phenol substituted at *para* position phosphorus (III) chloride in refluxing acetonitrile is subjected to amidoalkylation reaction with benzyl carbamate and an aldehyde such as acetaldehyde, propionic aldehyde, iso-butyric aldehyde, phenylacetic aldehyde, p-nitrobenzoic aldehyde and isovaleric aldehyde, is followed by the synthesis of hydrobromide salts of 1-aminoalkylphosphonate diaryl esters, in which benzyloxycarbonyl (Cbz) protective group is removed, and in the next step is reacted with an amino acid containing protected α-amino group in the presence of a coupling reagent. The resulting phosphonic dipeptide containing tert-butoxycarbonyl (t-Boc) protective group is subjected to deprotection of α-amino group and obtained dipeptidyl 1-aminoalkylphosphonate diaryl ester is coupled with another amino acid with protected α-amino group, biotin or a derivative thereof in a manner analogous to that described above.

4. The method according to claim. 3 **characterized in that** the coupling reagent is used O-benzotriazol-1-yl-N, N, N ', N'-tetramethyl-uronium hexafluorophosphate (HBTU) in the presence of N, N'-diisopropylethylamine (DIEA).

5. Use of 1-aminoalkylphosphonate diaryl esters of formula I, in which Bt represents biotin, W is a hydrocarbon linker chain of 5 to 10 carbon atoms, Y is the amino acid residues in an amount of 0 to 4, R is a substituent corresponding to the side chain of valine, alanine, leucine, phenylalanine, 4-guanidinephenylglycine, or 2-aminobutyric acid (Abu), X is the hydrogen or a compound selected from the group consisting of mercaptomethyl (S-methyl), methoxy (O-methyl), or carboxymethyl as inhibitors of serine proteases.

6. Use of 1-aminoalkylphosphonate diaryl esters of formula I, in which Bt represents biotin, W is a hydrocarbon linker chain of 5 to 10 carbon atoms, Y is the amino acid residues in an amount of 0 to 4, R is a substituent corresponding to the side chain of valine, alanine, leucine, phenylalanine, 4-guanidinephenylglycine, or 2-aminobutyric acid (Abu), X is the hydrogen or a compound selected from the group consisting of mercaptomethyl (S-methyl), methoxy (O-methyl), or carboxymethyl as the low molecular weight molecular probes for detection of catalytically active enzyme forms.
